(19) Europäisches Patentamt / European Patent Office / Office européen des brevets

(11) **EP 3 968 852 B1**

(12) **EUROPEAN PATENT SPECIFICATION**

(45) Date of publication and mention
of the grant of the patent:
**08.07.2026 Bulletin 2026/28**

(21) Application number: **20729956.1**

(22) Date of filing: **14.05.2020**

(51) International Patent Classification (IPC):
*A61B 5/0215* (2006.01)    *A61B 5/00* (2006.01)
*A61B 17/11* (2006.01)    *A61F 2/24* (2006.01)
*A61M 1/36* (2006.01)

(52) Cooperative Patent Classification (CPC):
**A61B 5/0215; A61B 5/686; A61B 5/6869;
A61B 17/11; A61F 2/064;** A61B 5/02158;
A61B 2017/00252; A61B 2017/1135; A61F 2/07;
A61F 2/966; A61F 2002/061

(86) International application number:
**PCT/EP2020/063553**

(87) International publication number:
**WO 2020/229636 (19.11.2020 Gazette 2020/47)**

(54) **A SHUNTING DEVICE**

RANGIERVORRICHTUNG

DISPOSITIF DE DÉRIVATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2019 US 201962847629 P**

(43) Date of publication of application:
**23.03.2022 Bulletin 2022/12**

(73) Proprietors:
• **O'Halloran, Tony**
**Co. Galway (IE)**
• **Thompson, John**
**Dublin 13, D13 K039 (IE)**
• **Balesh, Elie Rashid**
**El Paso, TX 79912 (US)**

(72) Inventors:
• **O'Halloran, Tony**
**Co. Galway (IE)**
• **Thompson, John**
**Dublin 13, D13 K039 (IE)**
• **Balesh, Elie Rashid**
**El Paso, TX 79912 (US)**

(74) Representative: **Purdylucey Intellectual Property**
**23 Ely Place**
**Dublin 2 D02 N285 (IE)**

(56) References cited:
US-A1- 2008 109 069    US-A1- 2017 113 026
US-A1- 2017 281 339    US-B1- 6 562 066

**Description**

Field of the Invention

The present invention relates to a shunting device.

Background to the Invention

Heart failure (HF) describes the complex clinical syndrome where the heart is incapable of maintaining a cardiac output (CO) that is adequate to meet metabolic requirements and accommodate venous return. There are multiple aetiologies leading to this final common clinical pathway, which carries a 50% 5-year mortality rate and is responsible for over one third of all deaths in the United States from cardiovascular causes. Worldwide, cardiovascular disease is on the rise and continues to be the leading cause of death. Each year, there are over 500,000 new cases in the United States and over 2 million new cases of HF diagnosed worldwide, leading to a prevalence of over 6 million in the United States and 30 million people across the globe.

Physiology of cardiac output: The amount of blood pumped by the heart over a given time period is known as cardiac output, which is in turn the product of HR and stroke volume (SV) and is typically 4-8 L/min. In addition, other factors such as synergistic ventricular contraction, ventricular wall integrity, and valvular competence all affect CO. SV is defined as the amount of blood ejected by the ventricle per heartbeat and is usually 1 cc/kg or approximately 60-100 cc. SV is affected by three main factors: preload, which is the amount of myocardial fiber stretch at the end of diastole; afterload, which is the resistance that must be overcome in order for the ventricle to eject blood; and contractility, which is the inotropic state of the heart independent of the preload or the afterload.

Types of heart failure:: Acute heart failure develops suddenly and symptoms are initially severe. Acute heart failure may follow a heart attack, which has caused damage to an area of your heart. It may also be caused by a sudden lack of ability by the body to compensate for chronic heart failure. If you develop acute heart failure, it may be severe initially, but may only last for a short period of time and improve rapidly. It usually requires treatment and medication to be administered by injection (intravenously). Chronic heart failure is very common. Symptoms appear slowly over time and gradually get worse. If symptoms, such as shortness of breath, get worse within a very short period of time in a patient with chronic heart failure, we call this an episode of acute decompensation. These episodes often need to be treated in hospital and should therefore be avoided. Left-sided heart failure means that the power of the left heart chamber, which pumps blood throughout the body, is reduced; thus, the left chamber must work harder to pump the same amount of blood.

There are two types of left-sided heart failure: Systolic failure: The left chamber lacks the force to push enough blood into circulation.

Diastolic failure: The left chamber fails to relax normally because the muscle has become stiffer and filling is impaired.

In right-sided heart failure, the right pumping chamber or ventricle, which pumps blood to the lungs, is compromised. This may be due to muscle injury, such as a heart attack localised to the right ventricle, damage to the valves in the right side of the heart or elevated pressure in the lungs. However, heart failure commonly affects both sides of the heart and is then called biventricular heart failure.

Pathophysiology of Left sided HF: There are two types of left-sided heart failure, systolic failure and diastolic failure. Systolic heart failure occurs when the contraction of the muscle wall of the left ventricle malfunctions, which compromises its pumping action. This causes a decrease in the ejection fraction below the normal range, and over time, enlargement of the ventricle. Diastolic heart failure occurs when the left ventricle muscle wall is unable to relax normally, because the muscle has become stiff. When this happens, the heart does not fill properly, although the ejection fraction usually remains within the normal range, the stroke volume is reduced. Regardless of the malfunction, left-sided heart failure leaves the heart unable to pump enough blood into the circulation to meet the body's demands, and increased pressure within the heart causes blood to backup in the pulmonary circulation, producing congestion in your lungs. Pulmonary congestion from left sided HF is the common mechanism precipitating worsening symptoms and acute decompensation. Studies with implantable hemodynamic pressure monitoring have shown improved outcomes and a decrease in HF hospitalization by titration of medications to control left atrial pressure.

Pathophysiology of Right sided HF: The most common cause of right ventricular (RV) failure is LV failure. As the RV fails, there is a similar increase in the amount of blood in the ventricle, which in turn leads to elevated right atrial pressure and increased pressure in the vena caval system which impairs venous drainage from the body. This leads to increased pressure in the liver, the gastrointestinal tract, and the lower extremities and to the clinical signs and symptoms of abdominal pain, hepatomegaly, and peripheral oedema.

Ejection fraction:: The term ejection fraction is used to describe the chambers' strength and ability to empty with each beat. It can be measured in many ways but usually with echocardiography. If the pumping ability of the main pumping chamber is reduced, it's often referred to as HFrEF or Heart Failure with reduced Ejection Fraction. If the primary problem is abnormal relaxation during diastole, which impairs filling, the term HFpEF, or Heart Failure with preserved Ejection Fraction is often used. There is often overlap between these conditions with both reduced emptying and filling.

Pathophysiology of HFpEF: Heart failure with HFpEF accounts for ~50% of heart failure diagnoses, yet the underlying pathophysiology and diagnostic criteria are poorly defined. Partly as a consequence, no medical treatment has yet shown convincing outcome benefit for patients with HFpEF. The clinical hallmark of HFpEF is exertional breathlessness, at least in part due to an abnormal increase in left atrial pressure (LAP) during exercise. There are many potential mechanisms leading to reduced exercise tolerance in patients with HFpEF. In normal physiology, increased stroke volume during exercise is accomplished in part by the positive lusitropic consequence of sympathetic activation: left ventricular (LV) relaxation is enhanced with lower LV pressure in early diastole. Impaired LV relaxation and increased LV stiffness in patients with HFpEF prevents an increase in end diastolic LV volume during exercise, thus increasing pressure in the left atrium. The excessive increase in LAP, measured by pulmonary capillary wedge pressure (PCWP), during exercise is a common finding in patients with HFpEF and identifies those with a worse prognosis. Although patients with HFpEF reach a lower peak workload (watts per kilogram of body weight) during incremental exercise tests compared with normal controls, both reach similar peak exercise PCWP. Increased workload-indexed peak exercise PCWP may be diagnostic of early-stage HFpEF. A higher ratio of peak exercise PCWP to workload is associated with increased risk of 10-year mortality in patients with HFpEF.

Classification of HF:: Functional classification of HF generally relies on the New York Heart Association functional classification. The classes (I-IV) are:

> Class I: no limitation is experienced in any activities; there are no symptoms from ordinary activities.
> Class II: slight, mild limitation of activity; the person is comfortable at rest or with mild exertion.
> Class III: marked limitation of any activity; the person is comfortable only at rest.
> Class IV: any physical activity brings on discomfort and symptoms occur at rest.

This score documents the severity of symptoms and can be used to assess response to treatment.

Symptoms of HF: Exact diagnosis may be difficult since symptoms may be very similar, for example, all types of heart failure cause shortness of breath, fatigue and some degree of congestion, usually in the lungs but also in other parts of the body such as the liver, intestines, kidneys and lower limbs.

Management of HF: General measures for the treatment of HF include both lifestyle modification as well as medical therapies. Patients should be encouraged to lose excess weight, to abstain from tobacco and alcohol use, and to improve their physical condition through exercise as tolerated. Medical therapies include treatment of hypertension, dyslipidemia, diabetes, and arrhythmias, as well as sodium and water restriction. Revascularizable coronary artery disease should be treated as appropriate.

Pharmacologic management of HF: Pharmacologic management of HF includes many medications that are designed to counteract the deleterious effects of the compensatory mechanisms that have previously been discussed. Digoxin has been used to treat HF for over 200 years and acts to enhance inotropy of cardiac muscle and also reduces activation of the SNS and RAAS. Diuretics such as furosemide relieve fluid retention (pulmonary congestion and peripheral oedema) and improve exercise tolerance. ACE inhibitors such as captopril and enalapril block the conversion of angiotensin I to angiotensin II, which reduces activation of the RAAS. Angiotensin receptor blockers such as valsartan, losartan, and candesartan are used in patients who cannot tolerate ACE inhibitor therapy and work directly on the angiotensin receptors that are the final downstream target of the RAAS pathway. $\beta$-Blocking agents such as carvedilol and metoprolol are used to protect the heart and vasculature from the deleterious effects of overstimulation of the SNS and to help slow the heart down to allow for more efficient contraction. Aldosterone antagonists such as spironolactone also directly inhibit the RAAS. Inotropic agents such as milrinone provide direct stimulation of myocardium to increase contractility.

Surgical management of HF: Surgical management includes cardiac resynchronization therapy (CRT), coronary revascularization, surgical ventricular remodeling (SVR), ventricular assist device (VAD) implantation, and heart transplantation. Reversible ischemic heart disease can provide more functional myocardium and improve pump efficiency. CRT aims to improve ventricular efficiency by simultaneously pacing both ventricles. SVR attempts to surgically restore the normal geometry of the ventricle. VAD augments the decreased CO in HF, and heart transplantation replaces the failing heart with a new functional organ.

Implantable medical devices used in the management of HF: Implantable devices, such as cardiac resynchronisation therapy (CRT), improve symptoms and life expectancy in a subset of patients with heart failure and reduced ejection fraction (HFrEF). Some others allow early adjustment of medical therapy to avoid hospitalizations by monitoring cardiac haemodynamics. However, trials exploring the role of implantable devices such as CRT and rate-adaptive pacing in patients with HFpEF have stalled, mainly due to recruitment failure, perhaps reflecting a reluctance of older patients with several comorbidities to participate.

HFpEF is associated with increases in Left atrial pressure: The clinical hallmark of HFpEF is exertional breathlessness, at least in part due to an abnormal increase in left atrial pressure (LAP) during exercise. In patients with severe pulmonary artery hypertension (PAH), creation of a right to left atrial shunt reduces right atrial and ventricular pressures and improves symptoms, possibly due to increased systemic oxygen delivery due to increased cardiac output despite increasing cyanosis. Decompressing the left atrium might similarly provide symptomatic and haemodynamic improvement in patients with HFpEF. Reducing LAP with a percutaneously delivered atrial septal device is a novel potential therapeutic strategy.

Benefits of left atrial decompression: Creating an interatrial shunt for left atrial decompression has been successfully applied with blade and balloon septostomy for patients with myocarditis or end-stage cardiomyopathy and intractable pulmonary oedema. More recently, percutaneously implantable permanent interatrial shunt devices have been developed for treating patients with chronic HF and have shown promising early clinical and hemodynamic results. Most reports have focused on patients with HF with preserved ejection fraction (HFpEF).

Novel implantable devices to reduce Left atrial pressure: Surgical and medical interventions that alter LAP might have a significant impact on symptoms and mortality in various cardiac pathologies. One example is that a device making invasive measurement of LAP as a guide for medical therapy in patients with HeFREF (n=40) was associated with reduced LAP, improved symptoms, and reduced rates of worsening symptoms requiring intravenous diuretic therapy. In an observational study of 5 patients with high LAP and lower right atrial pressure (RAP) as a result of congenital obstructive left heart defects, the creation of an interatrial communication alleviated left atrial hypertension and improved symptoms. Conversely, pulmonary oedema can develop in some patients secondary to dramatic increases in LAP following closure of an atrial septal defect (ASD).

A number of percutaneously implantable interarterial shunts have been described in the medical and patent literature. In short-term, small-size clinical trials, both types have been shown to be associated with improvements in symptoms, quality of life measurements, and exercise capacity. These shunts also have observed and theoretical drawbacks, which may limit their effectiveness and use. Percutaneous implantation of interatrial shunts generally requires transseptal catheterization immediately preceding shunt device insertion. The transseptal catheterization system is placed from an entrance site in the femoral vein, across the interatrial septum in the region of fossa ovalis (FO), which is the central and thinnest region of the interatrial septum. The FO in adults is typically 15-20 mm in its major axis dimension and may be up to 10 mm thick. LA chamber access may be achieved using a host of different techniques familiar to those skilled in the art, including but not limited to: needle puncture, stylet puncture, screw needle puncture, and radiofrequency ablation. The passageway between the two atria is dilated to facilitate passage of a shunt device having a desired orifice size. Dilation generally is accomplished by advancing a tapered sheath/dilator catheter system or inflation of an angioplasty type balloon across the FO.

This is the same general location where a congenital secundum atrial septal defect (ASD) would be located.

The V-Wave device is a tri-leaflet porcine tissue valve on an hourglass-shaped nickeltitanium frame. The device is deployed percutaneously via a sheath (14 F) in the femoral vein, with fluoroscopic and intracardiac or transesophageal echocardiographic guidance under general anesthetic. Following radiofrequency trans-septal puncture, the center of the hourglass (5 mm diameter) is placed across the fossa ovalis with the ends of the hourglass sitting in left and right atria securing the device in place. The left atrial orifice is lined with expanded polytetrafluoroethylene (ePTFE) designed to improve blood flow and restrict new tissue growth over the device. Blood flows from the left to right via the porcine valve which is designed to close when RAP exceeds 2 mmHg, thus preventing right to left shunting. After device implantation, patients require anticoagulation with warfarin or direct-acting oral anticoagulant (DOAC) for 3 months and with low-dose aspirin indefinitely.

Device insertion was associated with improved symptoms (NYHA III at baseline vs II at 6 months), functional capacity (6-minute walk test distance 281 m at baseline vs 617 m at 6 months), and a substantial drop in NTproBNP (2983 pg/mL at baseline vs 1334 pg/mL at 6 months).

The IASD (interatrial shunt device) system developed by Corvia Medical, Inc. This nitinol device is composed of a left and right atrial disc (19-mm outer diameter), with an 8-mm communication. The device is deployed percutaneously via a sheath (16 F) in the femoral vein, with fluoroscopic and intracardiac or transesophageal echocardiographic guidance. The device is deployed after transseptal puncture of the mid-fossa ovalis, positioning the delivery catheter into the left atrium and deploying the left atrial disc, retracting and apposing this disc to the atrial septum, verifying the right atrial location of the delivery catheter, then deploying the right atrial disc such that the device is secured across the atrial septum The IASD differs from the V-Wave device in three ways: first, the device does not incorporate valve tissue; second, the inter-atrial communication is larger (8mm diameter compared with 5 mm with the V-Wave device); third, the device is a bare metal and not coated with ePTFE. Instead, the left atrial side of the device is flush with the atrial tissue to reduce the risk of thrombus formation. The REDUCE LAP-HF trial was an openlabel, nonrandomized phase 1 study of the IASD in patients with HFpEF and raised PCWP (≥15 mm at rest or ≥25 mmHg during exercise). After 6 months, IASD implantation was associated with reduced PCWP at rest in 52% of patients (n=32) and reduced PCWP measured by right heart catheterization during supine bicycle exercise in 58% of patients (n=34). The device was associated with reduced rest or exercise PCWP in 71% of patients (n=42) and reduced rest and exercise PCWP in 39% of patients (n=23). However, hemodynamic testing in a subset of patients at 12 months (n=18) showed no difference in average rest or exercise PCWP or RAP between baseline, 6 months, and 12 months.

Of the 64 patients who had the device implanted, 3 patients (5%) died between 6- and 12-month follow-up; 1 death was due to stroke, 1 due to pneumonia, and in 1 the cause was undetermined. There were 17 HF hospitalizations among 13 patients in the year post implantation, 10 of which occurred in 10 patients in the first 6 months. Left-right shunt through a patent device was confirmed on echocardiography at 12 months in all patients with good-quality images (n=48).

U.S. Patent No. 6,468,303 to Amplatz et al. describes a collapsible medical device and associated method for shunting selected organs and vessels. Amplatz describes that the device may be suitable to shunt a septal defect of a patient's heart, for example, by creating a shunt in the atrial septum of a neonate with hypoplastic left heart syndrome (HLHS). That patent also describes that increasing mixing of pulmonary and systemic venous blood improves oxygen saturation, and that the shunt may later be closed with an occluding device. Amplatz is silent on the treatment of HF or the reduction of left atrial pressure, or shunting to an vein or artery, as well as on means for regulating the rate of blood flow through the device.

U.S. Patent Publication No. 2005/0165344 to Dobak, III describes apparatus for treating heart failure that includes a tubular conduit having a emboli filter or valve, the device configured to be positioned in an opening in the atrial septum of the heart to allow flow from the left atrium into the right atrium. Dobak discloses that shunting of blood may reduce left atrial pressures, thereby preventing pulmonary edema and progressive left ventricular dysfunction, and reducing Left ventricular end diastolic pressure (LVEDP). Dobak describes that the device may include deployable retention struts, such as metallic arms that exert a slight force on the atrial septum on both sides and pinch or clamp the device to the septum. Dobak is silent on allowing flow from the left atrium to the Azygos vein.

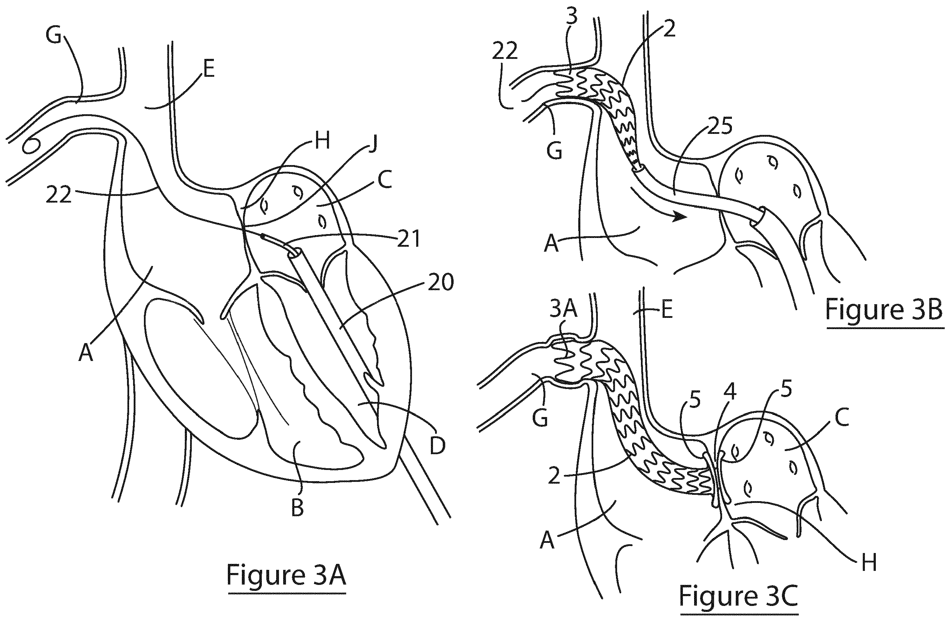

The atrial flow regulator (AFR):
Another example is the atrial flow regulator (AFR) developed by Occlutech International AB that is a nitinol mesh device composed of two flat discs and a 1- to 2-mm connecting neck with a central fenestration that enables bidirectional flow (Figure 3). It is manufactured in fenestration sizes of 6, 8, or 10 mm and is delivered via femoral venous approach with a 10- to 12-F sheath after an atrial septostomy.

The major advantage of these type of devices is the simplicity of manufacture. But these interatrial shunt devices have several important weaknesses that are anticipated to diminish their overall potential for clinical safety and effectiveness. However, the devices have a number of drawbacks

Susceptibility to occlusion of the shunt due to pannus infiltration:

A first drawback of these devices is the susceptibility to narrow or close during the postimplantation healing period. During the period following implantation, local trauma caused by crossing and dilating the FO, provoke a localized healing response, leading to neoendocardial tissue ingrowth, referred to as pannus. This tissue grows from the underlining tissue to cover the mesh and narrow or partially occlude the shunt orifice or impair the function of any valves within the device.

Shunt stenosis or occlusion occurred in one-half of the patients treated with an left to right atrial shunt by 1 year, as evaluated by trans oesophageal echo (TOE). The likely mechanism was found to be pannus infiltration of the bioprosthetic leaflets resulting in early valve degeneration. These patients who lost shunt function between serial echocardiographic examinations, then reverted to the natural history and progressive course of HF with increasing morbidity and mortality.

Although additional interventional cardiology procedures may be undertaken to restore lost luminal patency, such procedures may pose unacceptable risks, including death and stroke from embolization of the orifice-clogging material. There is also a risk of micro-emboli being sloughed off during this procedure, leading to silent brain infarcts and subsequent dementia.

Increased risk of paradoxical embolism:
A second drawback of these devices is the potential for paradoxical embolization.

Paradoxical embolization refers to thromboembolism originating in the venous vasculature (venous thromboembolism or VTE), such that an embolus traverses right-to-left through a cardiac shunt into the systemic arterial circulation. In normal circumstances, the pulmonary capillary bed acts as a filter, preventing venous embolic material from reaching the arterial circulation. However, right to left shunts allow emboli to cross into the arterial circulation without traversing the lungs. The most severe complication of paradoxical embolization occurs when an embolus lodges in the cerebral circulation with resulting cerebral infarction (stroke). Similarly, if a paradoxical embolus enters the coronary arterial circulation, myocardial infarction (MI) may ensue.

It has been asserted that in order for VTE to enter the systemic circulation, the prevailing LA to RA pressure gradient must be temporarily reduced, eliminated or reversed so that blood will either flow slowly across the shunt, cease to flow across the shunt or flow retrograde across the shunt. Echo/Doppler imaging studies often reveal some amount of shunting in both directions (bi-directional shunting) in patients with congenital ASD, even when LA to RA flow predominates. Bidirectional shunting may be best demonstrated when a subject performs a Valsalva manoeuvre (straining caused by exhalation against a closed glottis). However, this may be a simplification of the intra-atrial hemodynamics and it is possible that some blood may flow counter to the predominant direction of flow in the shunt.

Valsalva increases intrathoracic pressure, which causes the RA and LA pressures to equalize after several seconds and then for the RA pressure to transiently exceed LA pressure on exhalation. Intermittent bidirectional flow also may be observed at rest when the interatrial pressure gradient is low, or intermittently during the cardiac cycle when LA contraction is delayed compared to RA contraction (interatrial conduction delay). This is seen especially when the atria are enlarged or diseased, such as in heart failure. In this setting, interatrial electrical conduction delay results in retardation of LA contraction. Bidirectional shunting can also be seen transiently during inspiration, when venous return to the RA is increased, during coughing, with abdominal compression, during forced exhalation, or in the presence of severe tricuspid valve regurgitation. Chronically increased pulmonary arterial pressure, as seen in severe pulmonary hypertension, whether primary or secondary to chronic lung disease, recurrent pulmonary embolism, or due to chronic right ventricular volume overload, has been associated with chronic and more severe RA to LA shunting.

Migraines with Aura (MA) are very painful headaches. In people who have migraines with aura, these headaches cause blurry vision and blind spots. Some studies have linked Naturally occurring inter-atrial shunts with migraines and some patients have found that their migraine headaches go away after the shunt is closed. One possible mechanism of explaining how Naturally occurring inter-atrial shunts may play a role in MA is related to the occurrence of subclinical emboli and/or higher concentrations of serotonin and other metabolites that avoid the lungs and directly enter the systemic circulation. This causes irritation of the trigeminal nerve and brain vasculature, triggering migraine. Therefore, it is conceivable that artificially created inter-atrial shunts would hold a similar risk.

Interatrial shunt devices could also cause of silent brain infarcts. Silent infarcts are associated with subtle deficits and increase the risk of subsequent stroke and dementia by approximately two-fold. Compared to small vessel disease, silent brain infarcts associated with cardiac disease are underrecognized. Naturally occurring inter-atrial shunts are reportedly associated with silent brain infarcts. Therefore, it is conceivable that artificially created inter-atrial shunts would hold a similar risk.

Therefore, the risk of paradoxical emboli and silent brain infarcts due to localised reversal or transient reversal of the left-to-right shunt to a right-to-left shunt because of increase in right atrial pressure (for example, during a severe Valsalva manoeuvre) is real possibility. Right heart dysfunction and pulmonary hypertension with elevated pulmonary vascular resistance represented exclusion criteria in the IASD studies.

Increased risk of right heart failure:
Left-to-right shunts as described in the preceding technologies, which simply redistributes blood with the atria of the heart can ultimately lead to chronic right heart failure because of volume overload. The clinical significance of left-to-right shunts depends largely on their size and the volume of blood flow through them. As blood is shunted into the right atrium, this causes an increase in right ventricular filling, leading to an increase in right ventricular end diastolic volume and an increase in right ventricular end diastolic pressure. Over time this causes right ventricular hypertrophy, when right-sided pressures exceed left-sided pressures, the left-to-right shunt switches and becomes a right-to-left shunt. Dyspnea, fatigue, and cyanosis develop in a condition termed Eisenmenger syndrome.

Introduction of a left-right atrial shunt with the IASD was associated with an increase in the right ventricular volume and ejection fraction, but not RAP compared to baseline. There was no effect on LVEF and left atrial volume, although LV diastolic volume decreased.

A chronic left-right shunt increases pulmonary blood flow and may be well tolerated at younger ages: patients with ASD are at increased risk of PAH and atrial arrhythmias, but clinical problems do not usually emerge until the fourth or fifth decade. However, in elderly individuals (the average age of patients in REDUCE LAP-HF was 69 years) with HFpEF and altered ventricular compliance, left-right atrial shunts may cause increased pulmonary arterial pressure and subsequent right ventricular dysfunction. Such hemodynamic changes might affect other organs already compromised in HFpEF, such as the kidneys, with a subsequent negative impact on long-term outcome.

The requirement for long-term anti-thrombotic treatment:
Antithrombotic treatment is needed after implantation to prevent thromboembolic deviceassociated complications. Empirical treatments with oral anticoagulation for 3 months followed by ASA monotherapy lifelong after V-Wave implantation and dual antiplatelet therapy for 6 months followed by ASA monotherapy lifelong after IASD-implantation were used. Increase risk of bleedings during antithrombotic treatment has also to be taken into account in this frail population.

The inability to safely retrieve current devices once implanted:
Should the shunt become a nidus for infection, develop fatigue or corrosion fractures of its metallic framework, or erode or otherwise impinge on other vital cardiac structures, it cannot be removed by percutaneous retrieval/removal techniques. This is because the shunt, with its large "footprint" on the interatrial septum, is encased in pannus tissue.

Attempts at percutaneous removal may result in tearing of the septum, pericardia! tamponade, and device embolization into the systemic circulation, resulting in death or the need for emergency surgery. Safe removal would require performing open heart surgery.

This entails that the heart be bypassed using an extracorporeal membrane pump oxygenator (cardiopulmonary bypass), so the heart can be opened, the shunt removed, and the septum repaired. Performing such surgical procedures in patients with already established severe heart failure, including its frequently associated co-morbid conditions such as peripheral, cerebrovascular, and coronary artery disease, renal dysfunction, and diabetes, would be expected to have substantial risks for mortality or severe morbidity.

Current inter-atrial shunt devices require large access sheaths for deployment:

The IASD device is deployed percutaneously via a 16G sheath in the femoral vein and the V-wave device is deployed percutaneously via a 14F sheath in the femoral vein. Large access sheaths carry an increased risk of intra-cardiac complications including pericardial effusion and tamponade, thromboembolism, air embolism, persistent atrial septal defect and inadvertent puncture of the aorta may occur. There is also an increased risk of access site complications including hematomas and pseudoaneurysms.

A significant fraction of these chronic HF patients will develop chronic kidney disease or or end stage renal failure, for which the best management is hemodialysis via an upper extremity arteriovenous fistula/graft (AVG/AVF), ie a brachial artery to cephalic vein fistula. This creates a high flow circuit that increases RA pressure, rendering an inter-atrial shunt to decompress the LA useless and worsening LA pressures.

Also,

routinely interventional physicians will declot thrombosed AVFs/AVGs, and this can slough off clot which travels to lungs, this is a known risk of reopening the dialysis circuit. Having an inter- atrial shunt in this scenario would lead to an unacceptable risk of paradoxical emboli. Therefore, haemodialysis patients will likely be contraindicated from availing of the technology described previously.to our competitors' approaches which we avoid.

US6562066 describes a stent for arteriolization of the coronary sinus.

US2008/109069 describes a coronary sinus device to treat ischaemic heart disease via retrograde venous perfusion of myocardium wit oxygenated blood. The device does not alter left atrial pressure, but instead relies upon elevated left atrial pressure to create a large enough gradient to overcome the resistance to flow intrinsic to the low capacitance coronary sinus venous system.

US2017/281339 describes an isolated intracardiac shunting device to treat restrictive heart disease by reducing left atrial pressure. A problem with this device is that while it addresses pressure imbalances in the left atrium, it results in the pressure in the right side of the heart to be altered.

It is an object of the invention to overcome at least one of the above-referenced problems.

## Summary of the Invention

The present invention addresses one or more of the problems of the implantable devices of the prior art by providing an implantable shunting device as set out in the claims configured to shunt blood from the left atrium to the azygos vein through an aperture in the atrial septal wall. The azygos vein transports deoxygenated blood from the posterior walls of the thorax and abdomen into the superior vena cava vein. The device enables redistribution of left atrial blood volumes and pressure imbalances away from the heart reducing the risk of right heart failure and the risk of paradoxical emboli entering the left side of the heart, and in addition provides a more durable configuration that maintains luminal patency for extended periods of time. The device is configured for delivery via a small access sheath and is fully retrievable.

In one embodiment the device comprises a sensor configured to detect a parameter of the heart or blood in the heart, for example left atrial or right atrial blood pressure, and transmit data relating to the parameter to an external location for display. The device may include two sensors, one configured to detect a right atrial parameter (such as right atrial blood pressure) and one configured to detect a left atrial parameter (for example left atrial blood pressure), and transmit data relating to the detected parameters to an external receiver. The ability to monitor atrial pressure(s) can be used to monitor the effectiveness of the shunting device and/or monitor pressure imbalances in the heart (for example early detection of left atrial pressure drop or left atrial hypertension). Having a sensor in the right atrium allows early detection of right atrial hypertension and better diagnosis of left to right pressure imbalances. In addition, as the accuracy of sensors reduces over time (so called "drift"), the sensors have to be calibrated. Calibrating a left atrial sensor is problematical due to the inaccessibility of the left atrium, however when the device also has a right atrial sensor (which is more easily accessible), it is possible to calibrate both left and right atrial sensors based on the calibration data from the right atrial sensor, as the drift of both sensors will be the substantially the same.

The device may also include a valve configured to control flow of blood through the device. The valve may be configured for self-actuation in response to pressure changes in the blood. The valve may be configured for retro-fitting to the shunting device, optionally in-vivo retro-fitting. This allows a shunting device with one or more sensors to be implanted into a patients heart, and heart parameter data (for example left and/or right atrial pressures) to be measured and/or recorded during a period of implantation. The heart parameter data (for example left and/or right atrial blood pressure data) may be used to design a valve for the shunting device that is specifically tailored to control pressure imbalances in the patient detected during the period of implantation, and the patient-specific valve may then be retro-fitted to the shunting device.

The valve may be configured to actuate in response to data from the sensor. The device may include a controller configured to receive data from the or each sensor and actuate the valve in response to the received data. For example, if the sensor detects a left atrial blood pressure below a defined threshold, the controller may actuate the valve to limit or stop left to right blood flow through the device. Likewise, if the sensor detects a left atrial blood pressure above a defined threshold, the controller may open the valve to increase left to right blood flow through the device. The controller may be part of the shunting device and implantable, in which case it is generally operatively connected to the sensor(s) and valve. The controller may also be configured for location remote to the device (for example outside of the body) and communication (for example wireless communication and/or wireless charging) with the sensor(s) and valve.

In a first aspect, the invention provides an implantable shunting device according to Claim 1.

The tube is typically flexible. This allow the tube bend to traverse from the atrial septal wall through the right atrium and up into the inferior vena cava, and typically curve into the ostium of the azygos vein. The tube may be provided as a single unitary tube, or as two or more tubes configured for engagement in-situ in the heart to form a single tube. In other embodiments described below, the tube may be provided in a modular format. The tube may comprise straight sections configured to assemble in-situ to provide a tube which traverses from the left atrial wall to the ostium of the azygos vein. The straight sections are typically not axially flexible.

In one embodiment, the device has an axial length of about 2.5 to 4.5 cm, preferably about 3.5 to 4.0 cm. In one embodiment, the conduit has a diameter of about 8 to 15 mm, preferably about 9 to 11 mm. It will be appreciated that the length and diameter of the conduit can be tailored to suit an individual patient. In one embodiment, the device will be chosen based on an initial imaging of the patient which will help determine the optimum length and diameter of the device.

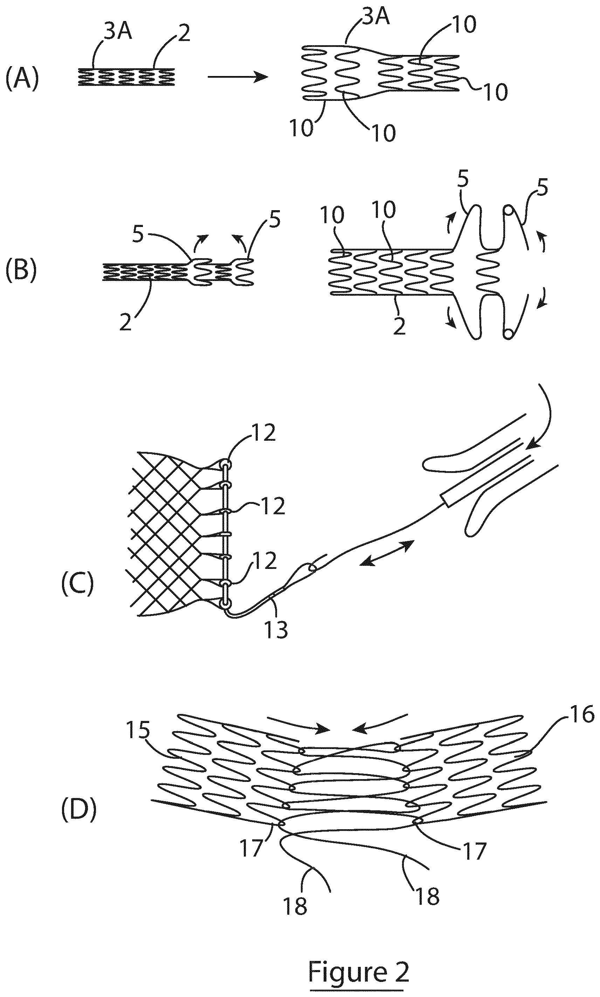

In one embodiment, the distal end is configured for over-expansion to anchor the distal end in an ostium of the azygos vein (Fig. 2). In one embodiment, the diameter of the overexpanded distal end is about 5-25% greater upon deployment that the diameter of the conduit part of the device. It will be appreciated that the diameter of the distal end upon deployment will be chosen to suit the anatomy of the patient, and can be determined prior to implantation by means of imaging.

In one embodiment, the proximal end comprises two axially spaced apart expansible retention flange sections configured for expansion on each side of an atrial septal wall to anchor the distal end of the device (Fig. 3). In one embodiment, the diameter of the retention flanges on deployment is between 10 and 25 mm. Typically, the retention flanges are axially spaced apart by a distance approximately equal to a thickness of the atrial septal wall where the aperture is located. In this manner, the retention flanges upon deployment anchor the device to the atrial septal wall. Examples of atrial septal wall anchors of this type are described in US6,468,303.

In one embodiment, the device is self-expansible (i.e. upon retraction of a delivery catheter/sheath, the device self-expands to a deployed configuration). This, the device may comprise a nitinol material, for example super elastic NITi. Typically, the distal end is configured to self-expand to a diameter greater than the diameter of the azygos vein (i.e. the ostium of the azygos vein) to anchor the proximal end in the azygos vein. Typically, the distal retention flanges are configured to self-expand to a diameter greater than the diameter of the aperture in the atrial septal wall.

In another embodiment, the device is not self-expanding, for example it may be formed from a material that is not self-expanding, such as stainless steel. In this embodiment, the device may be deployed using an expansion member such as a balloon.

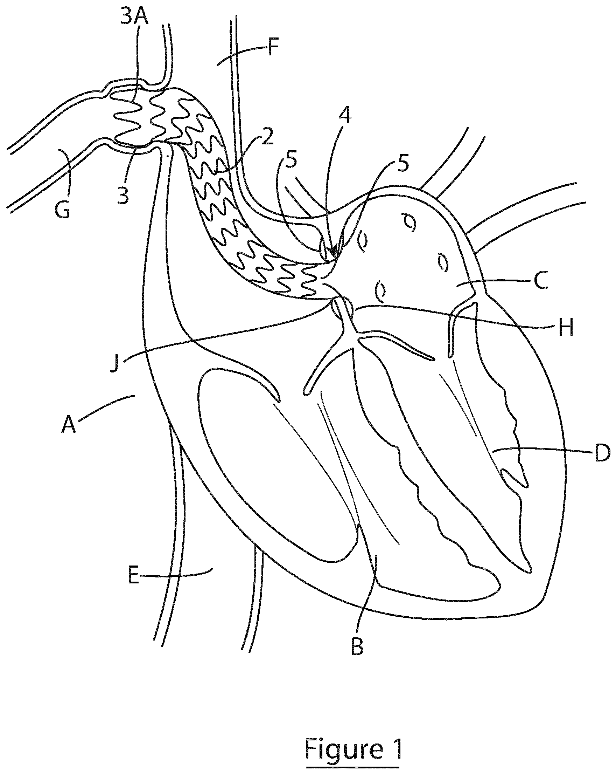

In one embodiment, the device is pre-formed to assume a curved shape (such as the curved shape shown in Fig. 1) upon deployment.

In one embodiment, the device comprises a valve. The valve may be, for example, configured to control right to left blood flow (for example to actuate when the right side pressure exceeds a pre-defined threshold pressure). It may also be configured to control left to right blood flow (for example when the left side pressure drops below a pre-defined threshold pressure). In one embodiment, the valve comprises a TPU material or animal derived pericardium (ovine, porcine, or bovine, for example), and may be produced by reaction moulding. The valve is attached to the structural wire element, generally. In one embodiment, the valve is configured to prevent thrombus passing right to left. The valve may be configured for retro-fitting to the shunting device in-vivo or ex-vivo.

In one embodiment, the device comprises a sensor, typically configured to detect a parameter of blood passing through the device or tissue in the heart. Examples of parameters include temperature, pressure, pH, blood flow, impedance, electrical conductivity. The sensor may be an optical sensor. In one embodiment, the sensor (or the device) comprises a wireless communication module configured to wirelessly send signals from the sensor to a remote location (for example a remote device comprising a receiver for the signals and display). The sensor may be configured to detect a parameter of blood or tissue in right atrium or left atrium. In one embodiment, the sensor is configured to detect a parameter of blood in the left atrium. In one embodiment, the sensor is configured to detect a parameter of blood in the right atrium. In one embodiment, the device comprises two sensors. In one embodiment, one of the sensors is configured to detect a parameter of blood in the left atrium and another of the sensor is configured to detect a parameter of blood in the right atrium. In one embodiment, the parameter is pressure. The sensor for detecting a parameter of left atrial blood may be disposed on the shunting device and project into or adjacent to the left atrium. The left atrial sensor may be disposed at least partly within the lumen of the shunting device, and be configured to measure a parameter of blood in the shunting device (this data may be correlated with left atrial pressure using a suitable algorithm). The sensor for detecting a parameter of right atrial blood is generally disposed at least partly in the right atrium, and/or may be disposed on an external surface of the shunting device in the right atrium.

The device may comprise an energy storage module operatively connected to the or each sensor and optionally a valve. Typically the energy storage module is configured for wireless charging (which allows the battery to be re-charged while the device is in the heart).

The valve may be configured for actuation in response to data received from the or each sensor. For example, a controller may be provided and configured to receive data from the or each sensor and actuate the valve in response to the received data. Thus, the controller may be configured to actuate the valve to limit or reduce left to right blood flow if it detects that the left atrial blood pressure is lower than a threshold left atrial pressure or if the right atrial blood pressure exceeds a right atrial blood pressure. The controller may be provided as part of the device, or may be a remote controller configured for use outside of the body or remote to the body. The controller may be configured for wireless communication with the sensor(s) and/or valve. The controller may comprises a processor configured to compare data received from the sensor (for example left atrial blood pressure data) with reference data (for example reference left atrial blood pressure) and actuate the valve based on the comparison.

In one embodiment, the valve is configured for retro-fitting to the shunting device, especially in-vivo retro-fitting. This allows a shunting device with one or more sensors to be implanted into a patients heart, and heart parameter data (for example left and/or right atrial pressures) may be measured and/or recorded during a period of implantation. The heart parameter data (for example left and/or right atrial blood pressure data) may be used to design a valve for the shunting device that is specifically tailored to control pressure imbalances in the patient detected during the period of implantation, and the patient-specific valve may then be retro-fitted to the shunting device. Retro-fitting may be performed externally (by withdrawing the shunting device, retro-fitting the patient-specific valve to the device, and then re-implanting the device with the valve), or it may be performed in-vivo. The valve is preferably configured for retro-fitting to the shunting device in-vivo. The patient-specific valve may be configured to open in response to a high threshold left-side pressure (i.e. to reduce left side pressure and re-distribute pressure to the right side via the shunt), It may also be configured to close in response to a low threshold left side pressure, or a rights side threshold pressure. The period of pressure monitoring prior to retro-fitting the valve may be usefully employed to determine these threshold pressure or pressures.

In one embodiment, the device is provided in two or more parts configured for assembly in-vivo (i.e. two-part shunting device). For example, the device may comprise a first part comprising the flexible tube and the distal end, and a second part comprising or consisting of the proximal end, where free ends of the first and second parts are configured for engagement in-vivo. In this embodiment, the proximal end is generally anchored in the aperture in the atrial septal wall first, and then the second part is deployed and anchored to the azygos vein, and the free ends of the parts are then connected in the right atrium to form the assembled device. In another embodiment, the device may comprise a first part comprising the proximal end and a proximal section of the flexible tube (conduit) and a second part comprising the distal end and a distal section of the flexible tube (conduit), whereby free ends of the flexible tube sections are configured for engagement in-vivo. Various engagement means for the free ends of the first and second parts may be employed, for example friction fit ends, magnetic connectors, threaded connectors, suture clips, or re-entrant slot connectors. The ends may be configured to reversible or non-reversible engagement. In one embodiment, each free end of the tethering element comprises loop members and tethering elements configured for lacing between the loops such that when the tethering elements are pulled, the free ends are pulled towards each other and laced together to form a continuous tube.

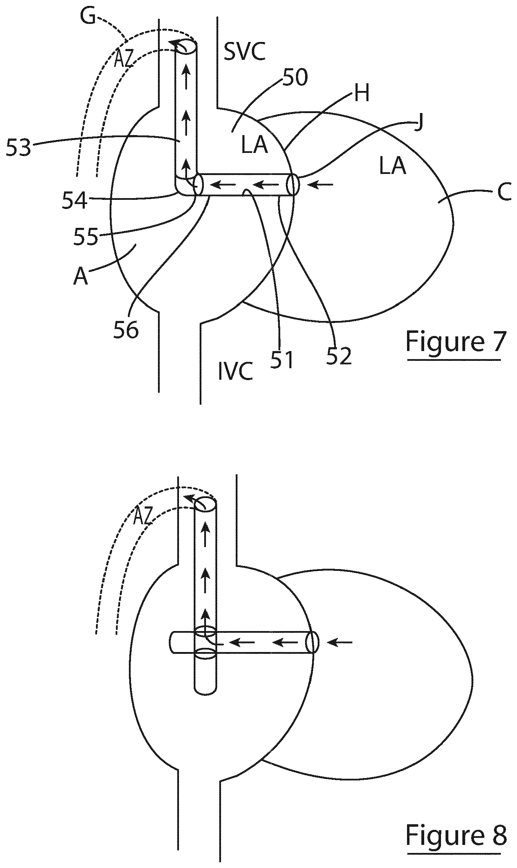

In another embodiment, the device is modular and comprises parts configured to fit together in-situ in the heart. The device may comprise 2, 3, 4 or more parts. The parts may comprise straight or curved conduits. The parts may be configured to be rigid upon deployment. In one embodiment, the parts are configured to fit together by friction fit or twist/lock means, or other mechanical engagement means. In one embodiment, one of the parts may comprise a proximal aperture configured to receive a distal end of another part. In one embodiment, the distal end is configured for radial expansion upon deployment in the proximal aperture to lock the two parts together and form a single conduit. In one embodiment, the parts are configured to inter-engage at right angles to each other. In one embodiment, the proximal end of one of the parts comprises opposed apertures configured to receive a proximal end of the other part, to provide fluid communication between the parts. These embodiments are illustrated in Figs 7 and 8.

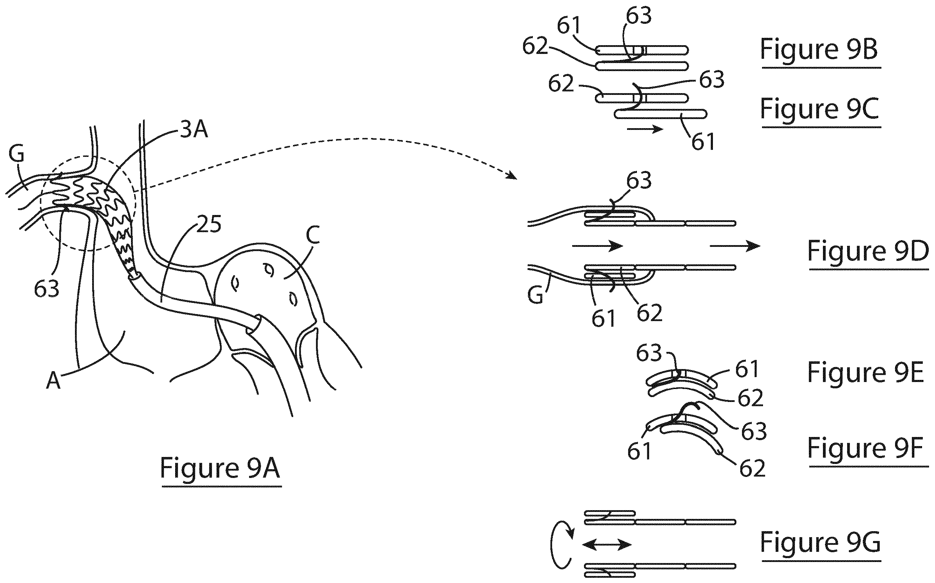

In one embodiment, the distal end of the device includes additional anchoring means, for example deployable hooks or barbs. In one embodiment, the distal end of the device includes an outer sleeve operatively connected to an inner sleeve for axial or rotational movement relative to the inner sleeve, and an anchoring element configured for radial extension/retraction upon movement (axial or rotational) of the outer sleeve relative to the inner sleeve. In one embodiment, the anchoring element is attached to the inner sleeve and projects through an aperture in the outer sleeve proximal of the attachment, such that proximal axial movement of the inner sleeve relative to the outer sleeve causes the anchoring element to project radially outwardly to anchor the device in the azygos vein. This is illustrated in Figs 9B-9D. In another embodiment, the anchoring means is configured for deployment of the anchoring means by rotation of one sleeve relative to the other. This is illustrated in Figs 9E to 9G. Typically, the anchoring element is a barb, ideally a curved barb.

The device may be formed from a tubular fabric or tubular mesh. Examples are described in detail in US6,4648,303, in particular columns 1 and 2, and US2017/0113026. Thus, the device may comprise a structural wire element typically formed from a metal (which may be a shape memory material) or a suitable polymer, and a biocompatible occluding sheath disposed on the inside or outside of the structural wire element. The wire element may be a single wire element (for example a helical wires) or may comprise a plurality of unconnected (or inter-connected) wire elements. The wires elements may be generally circumferential and include expansible sections along their circumference (as shown in Fig. 2). The structural wire element is configured to provide flexibility to the tube element of the device to allow it bend along its length without kinking and occluding the lumen of the device. Examples of structural wire elements configured for radial expansion in the body are well described in the cardiac stent field. The device may also be laser-cut device, or a 3-D printed device. The biocompatible occluding sheath or coating may be formed from polyethylene, TPU, PTFE stent encapsulation, or an electrospun material such as polyurethanes, urethan co-polymers, PET, or resorbable materials such as PLGA, PLLA, and PLA. The fibre size, material thickness, and fibre orientation can be configured as necessary.

The device may be self-expanding, for example upon retraction of a constraint such as a delivery sheath. Typically, the distal end is configured for self-expansion upon deployment from a delivery catheter to a diameter greater than the diameter of the azygos vein (preferably the diameter of the ostium of the azygos vein). Typically, the retention flanges of the proximal end of the device are configured for self-expansion upon deployment from the delivery catheter to a diameter greater than the diameter of the aperture in the atrial septal wall.

In one embodiment, the device is biodegradable, and typically configured for degrading in-vivo over a period of 8-15, 10-13, or 11-12 weeks. Examples of biodegradable stent materials will be known to a person skilled in the art and include Polydioxanon stent materials.

In one embodiment, the device is fully retractable and is configured to radial contraction and retraction into a removal catheter. In one embodiment, an end of the device may incorporate a face pull synch retraction mechanism that can be actuated to retract the device to a constrained configuration, prior to retraction of the device into a removal catheter and removal of the body. In one embodiment, an end of the device includes a series of loops and a tether threaded through the loops and configured such that pulling the tether causes the end of the device to radially contract.

In one embodiment, the device is configured for percutaneous delivery to the heart. In one embodiment, the device is configured for trans-apical delivery to the heart.

In one embodiment the invention provides a system comprising:

an implantable shunting device as set out in Claim 1, in which the shunting device comprises at least one sensor comprising a wireless communication module configured for wireless communication of data from the sensor; and

a remote device comprising a receiver configured to receive the data from the wireless communications module and a display for displaying the data.

In one embodiment, the remote device comprises a processor configured to compare data received from the sensor (for example left atrial blood pressure data) with reference data (for example reference left atrial blood pressure) and provide an output based on the comparison. In one embodiment, the reference data may be data from a second sensor on the device (for example, a sensor in the right atrium). The output may be a diagnosis of a disease, condition, or pathology, an indication of the functional status of the heart. The output may be displayed on the display.

In one embodiment, in which the implantable shunting device comprises a valve, the remote device may comprise a controller to actuate the valve in response to the data received by the receiver. Thus, the controller may be configured to actuate the valve to limit or reduce left to right blood flow if it detects that the left atrial blood pressure is lower than a threshold left atrial pressure or if the right atrial blood pressure exceeds a right atrial blood pressure.. The controller may be configured for wireless communication with the sensor(s) and/or valve.

In one embodiment, the controller is operably connected to the processor and configured to actuate the valve of the shunting device based on the output of the processor.

In one embodiment, the system comprises a wireless charging module for charging the energy storage module of the or each sensor.

In one embodiment, the invention provides a kit comprising an implantable shunting device as set out in Claim 1 and a delivery device configured for percutaneous or trans-apical delivery of the shunting device to a target location in the heart/vasculature. In one embodiment, the delivery device comprises a delivery catheter. In one embodiment, the delivery device comprises a guidewire, and the catheter is configured for over-wire percutaneous delivery to the heart or vasculature.

In one embodiment, the shunting catheter of the invention is a two-part device configured for assembly in-situ in the heart, typically the right atrium of the heart. In one embodiment, the delivery device comprises an outer sheath, two inner sheaths each providing a delivery lumen, wherein the outer sheath is axially adjustable relative to the inner sheath to deploy distal ends of the inner sheath in a bifurcated configuration (Fig. 12). Typically, one of the inner sheaths forming the bifurcated end of the delivery device is longer than the other. The first distal end of the inner sheath is typically dimensioned upon deployment to extend into the superior vena cava and terminate adjacent the ostium of the azygos vein. The second distal end of the inner sheath is dimensioned upon deployment to extend towards and terminate adjacent the atrial septal wall. In one embodiment, the delivery catheter is configured for delivery to the right atrium of the heart via a femoral and inferior vena cava approach. The use of this delivery device is described in more details below.

The implantable shunting device of the invention may be used to provide a conduit for blood flow from the left atrium through an aperture in the atrial septal wall to the azygos vein.

The implantable shunting device of the invention may be used to redistribute left atrial blood volume or correct or reduce left atrial blood pressure.

The implantable shunting device of the invention may be used to treat a disease or condition characterised by dysregulated (typically elevated) left atrial blood pressure or to treat or prevent heart failure or to treat or prevent conditions characterised by heart failure.

The implantable shunting device of the invention may be delivered to the heart of a subject by deploying a proximal end of the device to anchor the proximal end of the device to the azygos vein, and then deploying the second end of the device to anchor the distal end of the device to the atrial septal wall. The proximal end or distal end may be anchored first.

Delivery may include a step of making an aperture in an atrial septal wall. This may be performed percutaneously or trans-apically. Devices and methods for making apertures in the walls (wall puncturing devices) of the heart are known, and employ devices including wall puncturing needles, and energy delivery devices (i.e. tissue ablation electrodes). An aperture may be made in the wall, and then a balloon is positioned in the aperture and inflated to open the aperture.

The shunting device is delivered in a delivery catheter, and deployment of the device comprises retraction of a delivery catheter relative to the device to deploy the device.

Trans-apical Delivery

The shunting device may delivered trans-apically for example employing the steps of: trans-apically accessing the left ventricle;

advancing a delivery catheter containing the non-deployed shunting device into the ostium of the azygos vein via the left ventricle, left atrium, aperture in the atrial septal wall, right atrium and inferior cava; retracting the delivery catheter relative to the shunting device to deploy the proximal end of the shunting device to anchor the distal end in the azygos vein; further retracting the delivery catheter relative to the shunting device to deploy the tube; further retracting the delivery catheter relative to the shunting device to deploy a proximal retention flange on a right side of the atrial septal wall; and
further retracting the delivery catheter relative to the shunting device to deploy a distal retention flange on a left side of the atrial septal wall, to anchor the distal end of the device to the atrial septal wall, wherein the deployed and anchored device provides a conduit for blood flow from the left atrium to the azygos vein.

An aperture in the atrial septal wall may be created. This may be performed trans-apically or percutaneously, and may be generated using conventional methods, for example a wallpiercing needle, ultra-sonic probe, or tissue ablation electrode, the details of which will be known to the skilled person and will not be elaborated in more detail.

The left ventricle may be accessed transapically, a device advanced into the left atrium via the left ventricle, actuating the device to create an aperture in the atrial septal wall, advancing a guidewire into the azygos vein trans-apically via the left ventricle, left atrium, aperture in the atrial septal wall, and right atrium, and then advancing the delivery catheter over the guidewire to the azygos vein.

### Percutaneous delivery – IVC + aorta

The shunting device may be delivered percutaneously. The device may be delivered via an inferior vena cava approach, an aortic approach, or a combination of both.

When the shunting device is provided in two parts configured for engagement in-situ in the heart to form the assembled shunting device, delivery may involve:

delivering a first part to the heart and anchoring the first part (i.e. to the atrial septal wall); delivering a second part to the heart and anchoring the second part (i.e. to the azygos vein); and connecting the free ends of the two parts to provide fluid communication between the azygos vein and the left atrium.

When the first part is the proximal end, the proximal end may be delivered in a delivery catheter via the inferior vena cava to the right atrium, and the retention flanges of the proximal end deployed on each side of an aperture formed in the atrial septal wall.

When the second part comprises the distal end, delivery may involve delivering the second part in a delivery catheter via the aorta to the left atrium, through the proximal end anchored in the aperture in the atrial septal wall, and to the azygos vein via the right atrium, retracting the delivery catheter relative to the second part to deploy the second part with the distal end anchored in the ostium of the azygos vein, and connecting the free end of the second part to the proximal end anchored in the aperture in the atrial septal wall. Typically, the method includes an initial step of advancing a guidewire into the azygos vein via an aortic approach, and then advancing the delivery catheter over the guidewire to the azygos vein. Suitably, the step of advancing the guidewire comprises advancing a a guide sheath containing the guidewire to the azygos vein, and then retracting the guide sheath.

Percutaneous delivery - pre-existing shunt

When the subject being treated has an existing aperture in the atrial septal wall, and an existing shunt anchored in the aperture, the shunting device that is implanted into the subject may make use of the existing "shunt" and may be configured to fluidically connect to the existing shunt to form the assembled shunt of the invention. In this situation, the method comprises implanting a second part of the device into the heart of the patient, anchoring the distal end of the second part to the azygos vein, and connecting a free end of the second part to the pre-existing shunt to fluidically connect the left atrium and the azygos vein.

Percutaneous delivery - IVC and bifurcated delivery catheter

A delivery device may be employed that comprises an outer sheath, two inner delivery sheaths each providing a delivery lumen, wherein the outer sheath is axially adjustable relative to the inner delivery sheaths to deploy distal ends of the inner delivery sheaths in a bifurcated configuration. In one embodiment. Delivery may involve

advancing the delivery device into the left atrium via a femoral vein approach via the inferior vena cava;
retracting the outer sheath relative to the inner sheaths to deploy the distal end of the inner sheaths in a bifurcated configuration;
delivering the first part of the shunting device via a first of the inner sheaths; and

delivering a second part of the shunting device via a second of the inner sheaths.

When the distal ends of the inner sheaths are configured upon deployment to bifurcate with the first inner sheath projecting into the vena cava and the second inner sheath projecting towards the atrial septal wall, delivery may involve the steps of delivering the second part of the shunting device (comprising the proximal end of the shunting device) to the azygos vein through the first inner sheath, and delivering the first part of the shunting device (comprising the distal end of the shunting device) to the atrial septal wall through the second inner sheath.

Prior to delivery of the delivery device, a wall puncturing device may be delivered to the atrial septal wall of the left atrium through the second inner sheath, actuating the wall puncturing device to make an aperture in the atrial septal wall, and withdrawing the wall puncturing device.

Other aspects and preferred embodiments of the invention are defined and described in the other claims set out below.

Brief Description of the Figures

FIG. 1 is an illustration of a human heart in section showing a shunting device of the invention implanted in the heart and providing fluidic communication between the left ventricle of the heart and the azygos vein through an aperture formed in the atrial septal wall.

FIG.2A is an illustration of part of the shunting device of Fig. 1 showing the distal end of the shunting device in a constrained configuration (left side) and in a deployed, radially expanded, configuration (right side).

FIG.2B is an illustration of part of the shunting device of Fig. 1 showing the proximal end of the shunting device with retention flange sections in a constrained configuration (left side) and in a deployed, radially expanded, configuration (right side) .

FIG. 2C is an illustration of a face pull synch retraction mechanism forming part of the shunting device of the invention.

FIG. 2D illustrates part of a two-part shunting device according to the invention, having a first part, second part, each having a free end, and tethering elements that are laced between the sinusoidal ring struts at each free end.

FIG. 3 illustrates a trans-apical method of delivering and anchoring the shunting device of the invention.

**FIG. 4** illustrates a percutaneous method of delivering and anchoring a two-part shunting device of the invention via a combination of a femoral vein/IVC and aorta approach.

**FIG. 5** illustrates a bifurcated delivery device of the invention, and a percutaneous method of delivering and anchoring a two-part shunting device of the invention using the bifurcated delivery device via a femoral vein/IVC approach.

**FIG. 6** is an illustration of the venous architecture showing how the azygos vein can be accessed percutaneously via an approach through the common iliac vein and right ascending lumbar vein.

**FIG. 7** illustrates a modular shunting device according to the invention.

**FIG. 8** illustrates another modular shunting device according to the invention.

**FIG. 9** illustrates anchoring mechanisms of the invention: **(A)** the anchoring mechanism shown deployed in the azygos vein of the heart; **(B)** one embodiment of the anchoring mechanism showing the inner and outer tubes and the anchoring barb; **(C)** showing how axial movement of the inner sleeve relative to the inner sleeve causes the anchoring barb to extend radially outwardly; **(D)** similar to Fig. 9E, showing the anchoring the barb in the ostium of the azygos vein; **(E to G)** showing a second embodiment of the anchoring mechanism which is substantially the same as the anchoring system of Figs 9B-D with the exception that deployment involves rotational movement of the inner sleeve relative to the outer sleeve.

Detailed Description of the Invention

Definitions and general preferences

As used herein, the term "implantable shunting device" means a conduit configured to provide fluidic connection between the left atrium and the azygos vein, via an aperture in the atrial septal wall. The device may be employed to reduce fluid pressure in the left side of the heart, and thereby treat or prevent diseases or conditions characterised by elevated left side pressure. The device has a distal end configured to engage the azygos vein (generally at the ostium of the azygos vein) typically in a fluidically tight manner. In one embodiment, the proximal end of the device is configured for over-expansion in the ostium of the azygos vein, to anchor the end of the device in the vein and create a fluidically tight connection between the shunting device and the vein. The proximal end typically has a "shunt-like" end of the type known in the art configured to anchor to an atrial septal wall (See Figs 3A and 3B) having axially spaced-apart expansible retention flanges configured for deployment of each side of the wall to anchor to the wall, although other methods of anchoring to the atrial septal wall and establishing fluid connection with the left atrium via an aperture may be employed. The device is generally flexible and generally self-expansible, although non self-expansible devices that require expansion using a radial expansion device (i.e. a balloon) may be employed. The device (or at least the flexible tube part of the device) generally comprises a structural wire element (suitable for maintaining patency of the device) and a biocompatible occluding sheath (configured to prevent fluid leakage out of the device). The device upon deployment is generally sufficiently flexible to allow it to curve along its length (shown in Fig. 1), but it may also comprise a number of straight sections that are connected at an angle, or are hingedly connected, to provide a route from the aperture in the atrial septal wall to the azygos vein (as shown in Figs 7 and 8). The device may be delivered in an assembled form, or it may be delivered in parts and assembled in-situ in the heart.

As used herein, the term "azygos vein" refers to the part of the pulmonary venous system that transports deoxygenated blood from the posterior walls of the thorax and abdomen into the superior vena cava vein. It is formed by the union of the ascending lumbar veins with the right subcostal veins at the level of the 12th thoracic vertebra, ascending in the posterior mediastinum, and arching over the right main bronchus posteriorly at the root of the right lung to join the superior vena cava. A major tributary is the hemiazygos vein, a similar structure on the opposite side of the vertebral column. Other tributaries include the bronchial veins, pericardial veins, and posterior right intercostal veins. It communicates with the vertebrael venous plexuses. Accessing the azygos vein may be achieved by insertion of a catheter into the femoral vein, in a sizable subset of patients, the right ascending lumbar (RAL) vein anastomoses with the right common iliac vein, and in patients with hypervolemic states (ie HF), it will be more robustly formed. Advance the catheter into the RAL vein which can be confirmed easily with contrast venography. Advance a wire up the RAL vein in the Azygos and eventually to the Azygos ostium, once through the ostium the catheter will enter the superior vena cava and then the right atrium of the heart.

As used herein, the term "two-part shunting device" refers to a shunting device that is provided in two parts which are configured to be connected in-situ in the heart to form an assembled shunting device. For example, the device may comprise a first part comprising the flexible tube and the distal end, and a second part comprising or consisting of the proximal end, where free ends of the first and second parts are configured for engagement in-vivo. In this embodiment, the proximal end is generally anchored in the aperture in the atrial septal wall first, and then the second part of deployed and to the azygos vein, and the parts are then connected in the right atrium to form the assembled device. In another embodiment, the device may comprise a first part comprising the proximal end and a proximal section of the flexible tube (conduit) and a second part comprising the distal end and a distal section of the flexible tube (conduit), whereby free ends of the flexible tube sections are configured for engagement in-vivo. Various engagement means for the free ends of the first and second parts may be employed, for example friction fit ends, magnetic connectors, threaded connectors, suture clips, or re-entrant slot connectors. The ends may be configured to reversible or non-reversible engagement.

As used herein, the term "structural wire element" refers to the structural skeleton of the device, which is generally configured to allow the device be sufficiently flexible to allow it traverse from the atrial septal wall to the azygos vein), yet maintain patency. The wire element may comprise a single wire element, or a plurality of wire elements which may be connected or un-connected. Suitable structural wire elements are described in the cardiac stent prior art (see e.g., US6,468,303, US2017/0113026 and US2018/0263766). In one embodiment, the wire element comprises a plurality radially expansible circumferential struts, axially arranged along the tube. The structural wire element may be formed from a metal, for example stainless steel or a shape memory material such as Nitinol, or from a polymer material which may be laser cut.

As used herein, the term "biocompatible occluding sheath" refers to the cover on the structural wire element that occludes the lumen of the wire element, and may be formed on the inside or outside of the wire element. The biocompatible occluding sheath or coating may be formed from polyethylene, TPU, PTFE stent encapsulation, or an electrospun material such as polyurethanes, urethan co-polymers, PET, or resorbable materials such as PLGA, PLLA, and PLA. The fibre size, material thickness, and fibre orientation can be configured as necessary.

As used herein, the term "Transluminal delivery" means delivery of the shunting device to a target site (for example the heart) heart through a body lumen, for example delivery through an artery or vein. It is generally carried out by an interventional cardiologist. In one embodiment, the device of the invention is advanced through an artery or vein to deliver the device to the right atrium of the hear.

As used herein, the term "transapical delivery" means delivery through a wall of the heart. This usually requires a cardiac surgeon, and may be performed by means of open-heart surgery, or by means of key-hole surgery with access though the rib-cage.

As used herein, the term "delivery device" refers to a device, generally a delivery catheter, having at least one lumen configured to receive the shunting device (or part of the shunting device) in a contracted configuration, transport the device to the heart either percutaneously or trans-apically, and deliver the device at a target location in the heart. In one embodiment, the delivery device is configured for retraction relative to the contained device to deploy the device out of a distal end of the delivery device.

"Energy delivering element" refers to a device configured to receive energy and direct the energy to the tissue, and ideally convert the energy to heat to heat the tissue causing collagen denaturation (tissue ablation). Tissue ablation devices are known to the skilled person, and operate on the basis of emitting thermal energy (heat or cold), microwave energy, radiofrequency energy, other types of energy suitable for ablation of tissue, or chemicals configured to ablate tissue. Tissue ablation devices are sold by ANGIODYNAMICS, including the STARBURST radiofrequency ablation systems, and ACCULIS microwave ABLATION SYSTEMS. In one embodiment, the tissue ablation device comprises an array of electrodes or electrical components typically configured to deliver heat to adjacent tissue. In one embodiment, one or more of the electrodes comprises at least one or two thermocouples in electrical communication with the electrode. In one embodiment, one or more of the electrodes are configured to deliver RF or microwave energy.

"Sensor" means an electrical sensor configured to detect an environmental parameter within or adjacent to the shunting device, for example blood flow, electrical signal activity, pressure, impedance, moisture or the like. The sensor may be configured to detect a parameter of blood or tissue in the left atrium or right atrium, or both. The sensor may include an emission sensor and a detection sensor that are suitably spaced apart. In one embodiment, the sensor is an electrode. In one embodiment, the sensor is configured to detect fluid flow. In one embodiment, the sensor is configured to detect electrical conductivity. In one embodiment, the sensor is configured to detect electrical impedance. In one embodiment, the sensor is configured to detect an acoustic signal. In one embodiment, the sensor is configured to detect an optical signal typically indicative of changes in blood flow in the surrounding tissue. In one embodiment, the sensor is configured to detect stretch. In one embodiment, the sensor is configured to detect moisture. In one embodiment, the sensor is configured for wireless transmission of a detected signal to a processor. Examples suitable sensor include optical sensors, radio frequency sensors, microwave sensors, sensors based on lower frequency electromagnetic waves (i.e. from DC to RF), radiofrequency waves (from RF to MW) and microwave sensors (GHz). In one embodiment, the device has two sensors, one to detect a parameter of the left atrium and one to detect a parameter of the right atrium

"Optical sensor" means a sensor configured to direct light at the tissue and measure reflected/transmitted light. These sensors are particularly sensitive for detecting changes in blood flow in adjacent tissue, and therefore suitable for detecting devascularisation of tissue such as the LAA. Examples include optical probes using pulse oximetry, photoplasmography, near-infrared spectroscopy, Contrast enhanced ultrasonography, diffuse correlation spectroscopy (DCS), transmittance or reflectance sensors, LED RGB, laser doppler flowometry, diffuse reflectance, fluorescence/autofluoresence, Near Infrared (NIR) imaging, diffuse correlation spectroscopy, and optical coherence tomography. An example of a photopeasmography sensor is a device that passes two wavelengths of light through the tissue to a photodetector which measures the changing absorbance at each of the wavelengths, allowing it to determine the absorbances due to the pulsing arterial blood alone, excluding venous blood, muscle, fat etc). Photoplesmography measures change in volume of a tissue caused by a heart beat which is detected by illuminating the tissue with the light from a single LED and then measuring the amount of light either reflected to a photodiode.

Exemplification

The invention will now be described with reference to specific Examples. The insertion and therapeutic methods are not encompassed by the wording of the claims.

These are merely exemplary and for illustrative purposes only: they are not intended to be limiting in any way to the scope of the claimed invention which is defined by the appended claims.

Referring to the drawings, and initially to Figs 1 to 2, there is illustrated a human heart having a right atrium A, right ventricle B, left atrium C and left ventricle D, inferior vena cava E, superior vena cava F, azygos vein G, and atrial septal wall H. A shunting device of the invention, indicated generally by the reference numeral 1, is shown implanted in the heart A providing fluid communication between the left atrium C and azygos vein G though an aperture J formed in the atrial septal wall.

The shunting device 1 comprising a flexible tube 2 configured for radial adjustment between a contracted delivery configuration suitable for delivery in a delivery catheter and a deployed radially expanded configuration, the tube having a through lumen, a distal end 3 configured to anchor within the azygos vein G, and a proximal end 4 configured to span an aperture in an atrial septal wall and anchor to the wall. The deice has length of about X cm and diameter (along the flexible tube) of approximately Y cm, when deployed. The distal end 3 has an over-expansion section 3A to anchor within the ostium of the azygos vein having a diameter when expanded of about Z cm. The proximal end 4 comprises two axially spaced apart expansible retention flange sections 5 configured for expansion on each side of an atrial septal wall H to a diameter of approximately X cm to anchor the distal end of the device to the wall and establish fluidic connection between the device 1 and left atrium C via the aperture J.

As illustrated in Figs 2A and 2B, the device illustrated is self-expansible, and is formed from a structural wire element comprising a plurality of sinusoidal ring elements 10 configured for radial expansion from the constrained configuration shown in Fig 2A (left) to the unconstrained (deployed) configuration shown in Fig. 2A (right). The wire elements comprise a shape memory metal, such as NITI. The device also includes an occluding sheath covering the structural wire element and formed of polyethylene.

In more detail, and as illustrated in Fig. 2A, the distal end of the device is configured for engagement with the azygos vein, and in this embodiment comprise a self-expansible over-expansion section 3A having a diameter when deployed that is greater than the diameter of the ostium of the azygos vein. Referring to Fig. 2B, the proximal end of the device takes the form of a "shunt" and has two radially expansible retention flange sections axially separated by a distance of about X cm, and shown in a constrained configuration (left side) and deployed, radially expanded, configuration (right side). The flanges are dimensioned such that on deployment, they abut opposite sides of the atrial septal wall in an apposing relationship, anchoring the proximal end of the device to the wall.

As illustrated in Fig. 2C, the end of the device may incorporate a face pull synch retraction mechanism that can be actuated to retract the device to a constrained configuration, prior to retraction of the device into a removal catheter and removal of the body. In the embodiment shown, the end of the device includes a series of loops 12 and a tether 13 threaded through the loops and configured such that pulling the tether causes the end of the device to radially contract.

Figure 2D illustrates part of a two-part shunting device according to the invention, having a first part 15, second part 16, each having a free end 17, and tethering elements 18 that are laced between the sinusoidal ring struts 10 at each free end. When the tethering elements 18 are pulled, the free ends 17 are pulled towards each other and laced together to form a continuous tube.

Figure 3 illustrates a trans-apical method of delivery and implantation of a shunting device in the heart. In a first step, shown in 3A, the wall of the left ventricle D is punctured using a suitable puncturing device, and a catheter 20 is advanced through the hole and into the left atrium C via the left ventricle. A puncturing device (not shown) is then advanced through the catheter and actuated to form an aperture J in the atrial septal wall H. A guide sheath 21 containing a guidewire 22 is then advanced through the catheter 20, through the aperture J, the right atrium B, superior vena cava E, and into the azygos vein G. The guidewire is then deployed, and the guide sheath is retracted leaving the guidewire 22 in-situ. As shown in Fig, 3B, a delivery catheter 25 is then advanced along the same route over the guidewire 22 into the azygos vein, where the distal end 3 of the shunting device is deployed in the ostium of the azygos vein, where it expands into contact with the ostium of the azygos vein anchoring the distal end of the device in the vein. Deployment of the shunting device continues by retraction of the catheter 25 relative to the device 1, until the proximal end of the device is deployed as shown in Fig. 3C. This is generally performed using a cardiac imaging technique, such as fluoroscopy.

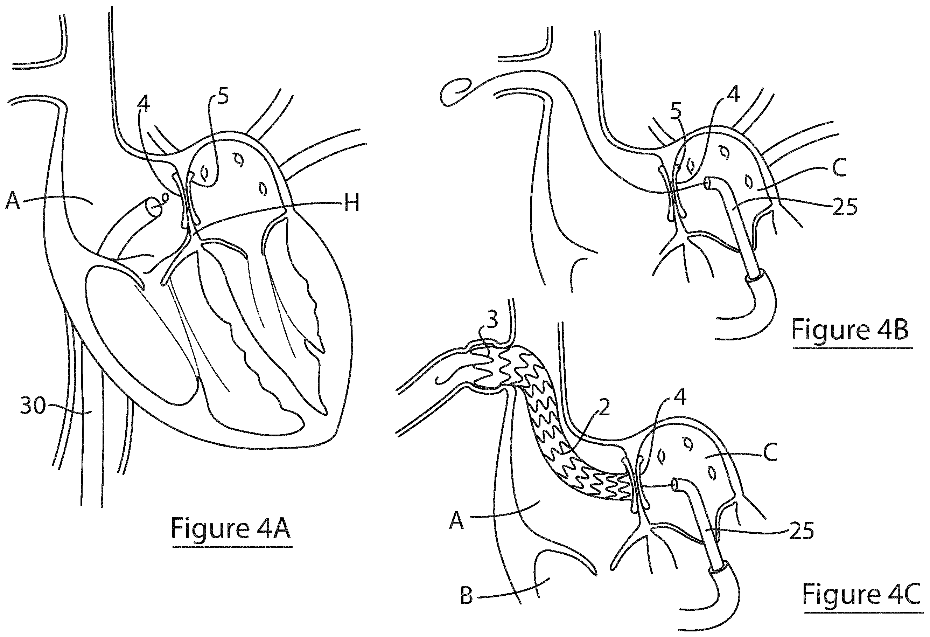

Figure 4 illustrates a fully percutaneous method of delivery and implantation of a two-part shunting device in the heart, in which steps described with reference to the previous embodiments are assigned the same reference numerals. In a first step, shown in Figure 4A, a delivery catheter 30 containing the first part of the shunting device (the proximal end 4 with retention flange sections 5) is advanced percutaneously via a femoral vein/IVC approach into the right atrium A and towards and across the atrial septal wall H (where an aperture has previously been formed using the techniques described previously). The proximal end 4 is then deployed across the aperture, such that the retention flanges self-expand upon deployment on each side of the wall, anchoring the proximal end 4 to the wall. Fig. 4B illustrates the delivery of the second part of the two-part device (which in this embodiment comprises the distal end 3 and flexible tube 2) percutaneously to the left ventricle via the aorta. The delivery steps are substantially the same as that described with reference to Figs 3A to 3C, with the exception that the delivery catheter is advanced into the right atrium through the lumen in the anchored proximal end 4, and that once deployed, the free end 15 of the tube 2 is connected to the anchored proximal end 4 as described previously with reference to Fig. 2D.

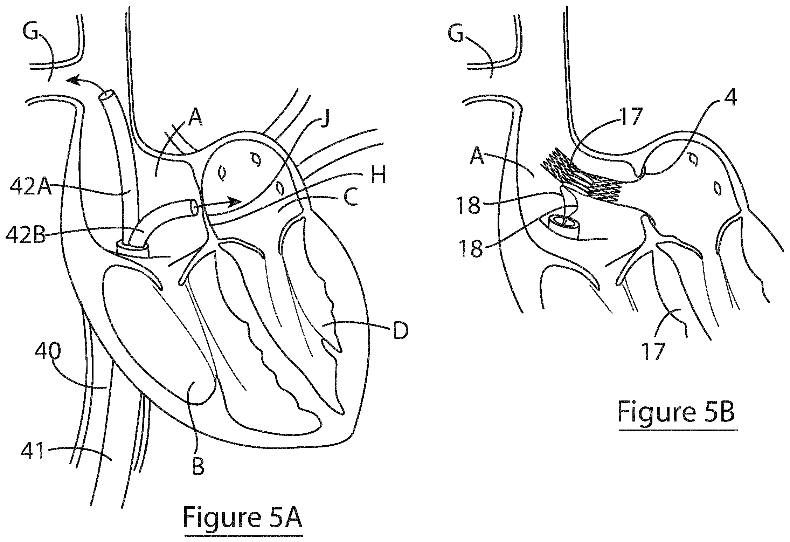

Figure 5 illustrates a delivery catheter, indicated generally by the reference numeral 40, and for use in delivering and implanting, a two-part shunting device of the invention, to the heart of a subject. The delivery catheter 40 comprises an outer sheath 41, and two inner delivery sheath 42A, 42B, that are axially movable relative to the outer sheath 41, and configured such that on deployment the inner sheaths 42A and 42B assume a bifurcated configuration shown in Fig. 5A. The inner sheath 42A is longer that the sheath 42B, and is configured upon deployment and advancement to project into the superior vena cava F and into the azygos vein G. The inner sheath 42B is configured upon deployment to project towards the atrial septal wall H. In use, the catheter 40 is advanced into the heart via a femoral vein/IVC approach and into the right atrium B where the inner sheaths are deployed and assume the bifurcated configuration shown in Fig. 5A. An ablation catheter (not shown) may then be advanced along inner sheath 42B and actuated to form an aperture in the wall H, before being retracted. The first part of the device (including the proximal end 4) is then advanced along inner sheath 42B and deployed across the atrial septal wall H as described previously. The second part (including the distal end 3) is then advanced along inner sheath 42A into the ostium of the azygos vein and deployed as described previously. The free ends 17 of the two parts are then meshed together using tethering elements 18 as described previously to form the assembled and implanted shunting device that provides fluidic connection between the left atrium and azygos vein.

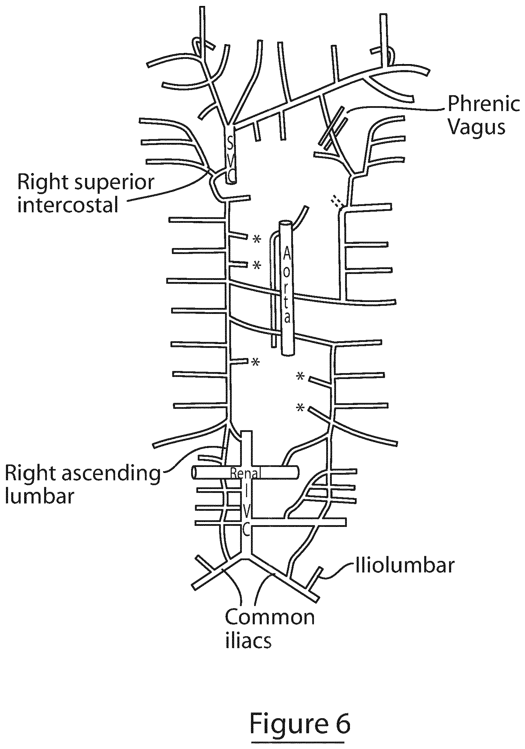

Figure 6 is an illustration of the venous architecture showing how the azygos vein can be accessed percutaneously via an approach through the common iliac vein and right ascending lumbar vein.

Referring to Figs 7 and 8, a modular device is illustrated, in which parts identified with reference to the previous embodiments are assigned the same reference numerals. In the embodiment of Fig. 7, the device 50 comprises a first tube 51 with a proximal end 52 configured to engage the atrial septal wall H at the aperture H, and a second tube 53 having a distal end 54 configured to anchor in the azygos vein G. The proximal end of the second tube 53 has an aperture 55 configured to receive a distal end 56 of the first tube 51 during deployment of the first tube, whereby radial expansion of the distal end of the first tube in the aperture 55 locks the two tubes together. The embodiment of Fig. 8 is similar to that of Fig. 7, with the exception that the distal end of the first tube comprises through apertures 58 configured to receive a distal end of the second tube 53. Both embodiments are configured for assembly in-situ in the heart to provide a conduit for blood flow from the left atrium to the azygos vein through the assembled device.

Referring to Figs 9, an additional anchoring means for the device, typically the distal end of the device is illustrated. The device of both embodiments comprises anchoring means (hooks or barbs) that are deployable by actuation of the distal end of the device. Fig. 9A shows the device anchored to an ostium of the azygos vein after the deployable anchoring elements have been deployed. In both embodiments, the distal end of the device includes an outer sleeve element 61 and an inner sleeve element 62, that are operatively coupled together and configured for relative axial movement (Fig. 9B and 9C) or relative rotational movement (Figs 9E and 9F). A curved anchoring barb 63 is attached to a distal end of the inner sleeve element 62 and is threaded through an aperture in the outer sleeve element 61 such that axial movement of the inner sleeve relative to the outer sleeve causes the barb to project outwardly into the ostium of the azygos vein (Figs 9B to 9D), or rotational movement of the inner sleeve relative to the outer sleeve causes the barb to project outwardly into the ostium of the azygos vein (Figs 9E to 9G).

The shunting device of the invention may be configured to detect blood pressure in the heart, for example in the left atrium and/or right atrium. Providing one or more sensors on the shunting device enables atrial pressures to be monitored which provides information of the effectiveness of the shunting device as well as early and accurate detection of pressure imbalances in the heart (for example early detection of left atrial pressure drop or right atrial hypertension). In one embodiment, the device has a first blood pressure sensor disposed on the left atrial side of the device and positioned to monitor left atrial blood pressure, and a second blood pressure sensor disposed on the right atrial side of the device and positioned to measure right atrial blood pressure. The sensors may be CardioMEMS HF System from CardioMEMS (Atlanta, GA) that consists of a battery-free sensor that can continuously measure systolic, diastolic, and mean pressures. The sensors are configured to transmit blood pressure data wirelessly to a remote monitoring device having a wireless receiver and a display for displaying the blood pressure. The data may be transmitted to an online portal where the patient's cardiologist can check the readings collected by the sensors. The monitoring device can have a processor configured to process the data by comparing the data with reference data and providing an output relating to the effectiveness of the shunting treatment and/or diagnostic information relating the heart, or the design of a patient-specific retro-fittable valve for the shunting device which can be retro-fitted to the shunting device in-vivo or ex-vivo.

**Claims**

1. An implantable shunting device (1) configured to shunt blood from the left atrium (C) of the heart through an aperture (J) in the atrial septal wall (H), the device comprising a tube (2) configured for radial adjustment between a contracted delivery configuration suitable for delivery in a delivery catheter and a deployed radially expanded configuration, the tube having a through lumen, a distal end (3), and a proximal end (4) configured to span an aperture in an atrial septal wall and anchor to the wall, **characterised in that** the distal end (3) of the tube is configured to anchor within the azygos vein and engage the azygos vein in a fluidically tight manner such that the device (1) is configured to shunt blood from the left atrium (C) of the heart to the azygos vein (G).

2. An implantable shunting device (1) according to Claim 1, in which the distal end (3) of the device is configured for over-expansion in the ostium of the azygos vein, to anchor the distal end of the device in the ostium of the azygos vein and create a fluidically tight connection between the shunting device and the azygos vein.

3. An implantable shunting device (1) according to Claim 1 or 2 in which the tube (2) is flexible and comprises a structural wire element suitable for maintaining patency of the device and a biocompatible occluding sheath configured to prevent fluid leakage out of the device.

4. An implantable shunting device according to any preceding Claim, in which the device comprises a sensor to detect a parameter of blood within or adjacent to the shunting device, in which the sensor is optionally configured to detect blood pressure.

5. An implantable shunting device according to Claim 4, in which the sensor comprises a wireless communication module configured to wirelessly send signals from the sensor to a remote location.

6. An implantable shunting device according to Claim 4 or 5, comprising a second sensor.

7. An implantable shunting device according to Claim 6, in which the sensor is configured to detect a parameter of blood in the left atrium and the second sensor is configured to detect a parameter of blood in the right atrium.

8. An implantable shunting device according to Claim 7, in which the sensor is configured to detect blood pressure in the left atrium and the second sensor is configured to detect blood pressure in the right atrium.

9. An implantable shunting device according to any preceding Claim, in which the device comprises a valve, in which the valve is optionally configured to control right to left or left to right blood flow, or passage of thrombus into the left atrium.

10. An implantable shunting device according to Claim 9, in which the valve is configured for retro-fitting to the shunting device in-vivo or ex-vivo.

11. An implantable shunting device according to any preceding Claim, in which the proximal end (4) comprises two axially spaced apart expansible retention flange sections (5) configured for expansion on each side of an atrial septal wall (H) to anchor the distal end of the device.

12. An implantable shunting device according to Claim 11, in which the retention flanges are axially spaced apart art by a distance approximately equal to a thickness of the atrial septal wall (H) where the aperture (J) is located.

13. An implantable shunting device according to any preceding Claim, in which the device is self-expansible.

14. An implantable shunting device according to Claim 1, in which:

    the device is pre-formed to assume a curved shape upon deployment or
    the device is modular and provided in two or more parts (15, 16) configured for assembly in-situ in the heart; or

the device comprises a first part comprising or consisting essentially of the distal end (3), and a second part comprising or consisting of the proximal end (4), wherein free ends (17) of the first and second parts are configured for engagement in-situ in the heart, wherein the first part optionally comprises the distal end (3) and the flexible tube (2), and the second part optionally consist essentially of the proximal end (4), or wherein the first part optionally comprises the distal end (3) and a first section of the flexible tube (2), and the second part optionally comprises the proximal end (4) and a second section of the flexible tube (2), or wherein free ends (17) of the first and second sections of the flexible tube are optionally configured for engagement in-situ in the heart; or the device comprises a structural wire element (10) and a biocompatible occluding sheath disposed on the inside or outside of the structural wire element, wherein the structural wire element comprises a shape-memory material or wherein the structural wire element comprises a plurality of circumferential and radially expansible wire struts (10); or the device is biodegradable; or the device is configured for radial contraction and retraction into a removal catheter; or a distal end (3) of the device comprises an anchoring mechanism comprising an outer sleeve (61) operatively connected to an inner sleeve (62) for axial or rotational movement relative to the inner sleeve, and a radially extendable anchoring element (63) associated with the outer and inner sleeve such that axial or rotational movement of the outer sleeve relative to the inner sleeve causes the radially extendable anchoring element to extend radially outwardly, optionally in which the radially extendable anchoring element (63) is attached to the inner sleeve (62) at an attachment point, and the outer sleeve (61) includes an aperture for receipt of a distal end of the anchoring element, in which the aperture is located proximally of the attachment point.

15. A system comprising a shunting device according to any of Claims 4 to 8 and a remote device comprising a wireless receiver configured to receive data relating to the or each sensed parameter and a display for displaying the data and/or a delivery device (25, 30, 40) configured for percutaneous or trans-apical delivery of the shunting device (1) to a target location in the heart/vasculature.

**Patentansprüche**

1. Implantierbare Shunt-Vorrichtung (1), die so konfiguriert ist, dass sie Blut aus dem linken Atrium (C) des Herzens über eine Öffnung (J) im Atriumseptum (H) ableitet, wobei die Vorrichtung einen Schlauch (2) umfasst, der für eine radiale Anpassung zwischen einer für das Einführen in einem Einführkatheter geeigneten zusammengezogenen Einführkonfiguration und einer radial expandierten, entfalteten Konfiguration konfiguriert ist, wobei der Schlauch ein Durchgangslumen, ein distales Ende (3) und ein proximales Ende (4) aufweist, das so konfiguriert ist, dass es eine Öffnung in einem Atriumseptum umspannt und an der Wand verankert wird, **dadurch gekennzeichnet, dass** das distale Ende (3) des Schlauchs so konfiguriert ist, dass es in der Vena azygos verankert wird und auf fluiddichte Weise an der Vena azygos anliegt, so dass die Vorrichtung (1) so konfiguriert ist, dass sie Blut aus dem linken Atrium (C) des Herzes in die Vena azygos (G) ableitet.

2. Implantierbare Shunt-Vorrichtung (1) nach Anspruch 1, wobei das distale Ende (3) der Vorrichtung für ein übermäßiges Expandieren im Ostium der Vena azygos konfiguriert ist, so dass das distale Ende der Vorrichtung im Ostium der Vena azygos verankert wird und eine fluiddichte Verbindung zwischen der Shunt-Vorrichtung und der Vena azygos entsteht.

3. Implantierbare Shunt-Vorrichtung (1) nach Anspruch 1 oder 2, wobei der Schlauch (2) flexibel ist und ein Strukturelement aus Draht, das sich zum Aufrechterhalten einer Durchgängigkeit der Vorrichtung eignet, und eine biokompatible Verschlusshülse umfasst, die so konfiguriert ist, dass sie ein Austreten von Fluid aus der Vorrichtung verhindert.

4. Implantierbare Shunt-Vorrichtung nach einem der vorhergehenden Ansprüche, wobei die Vorrichtung einen Sensor zum Erfassen eines Parameters von Blut in oder an der Shunt-Vorrichtung umfasst, wobei der Sensor wahlweise so konfiguriert ist, dass er Blutdruck erfasst.

5. Implantierbare Shunt-Vorrichtung nach Anspruch 4, wobei der Sensor ein Drahtloskommunikationsmodul umfasst, das so konfiguriert ist, dass es drahtlos Signale vom Sensor zu einem entfernt gelegenen Ort sendet.

6. Implantierbare Shunt-Vorrichtung nach Anspruch 4 oder 5, die einen zweiten Sensor umfasst.

7. Implantierbare Shunt-Vorrichtung nach Anspruch 6, wobei der Sensor so konfiguriert ist, dass er einen Parameter von Blut im linken Atrium erfasst, und der zweite Sensor so konfiguriert ist, dass er einen Parameter von Blut im rechten Atrium erfasst.

8. Implantierbare Shunt-Vorrichtung nach Anspruch 7, wobei der Sensor so konfiguriert ist, dass er Blutdruck im linken Atrium erfasst, und der zweite Sensor so konfiguriert ist, dass er Blutdruck im rechten Atrium erfasst.

9. Implantierbare Shunt-Vorrichtung nach einem der vorhergehenden Ansprüche, wobei die Vorrichtung ein Ventil umfasst, wobei das Ventil wahlweise so konfiguriert ist, dass es einen Blutfluss von rechts nach links oder von links nach rechts oder ein Passieren eines Thrombus in das linke Atrium beeinflusst.

10. Implantierbare Shunt-Vorrichtung nach Anspruch 9, wobei das Ventil für einen nachträglichen Einbau in die Shunt-Vorrichtung in-vivo oder ex-vivo konfiguriert ist.

11. Implantierbare Shunt-Vorrichtung nach einem der vorhergehenden Ansprüche, wobei das proximale Ende (4) zwei axial beabstandete, expandierbare Halteflanschabschnitte (5) umfasst, die zum Expandieren auf jeder Seite eines Atriumseptums (H) konfiguriert sind, so dass das distale Ende der Vorrichtung verankert wird.

12. Implantierbare Shunt-Vorrichtung nach Anspruch 11, wobei die Halteflansche um eine Distanz axial beabstandet sind, die in etwa einer Dicke des Atriumseptums (H) an der Öffnung (J) entspricht.

13. Implantierbare Shunt-Vorrichtung nach einem der vorhergehenden Ansprüche, wobei die Vorrichtung selbstexpandierbar ist.

14. Implantierbare Shunt-Vorrichtung nach Anspruch 1, wobei:

die Vorrichtung so vorgeformt ist, dass sie beim Entfalten eine gekrümmte Form annimmt, oder die Vorrichtung modular ist und in zwei oder mehr Teilen (15, 16) bereitgestellt wird, die zum Zusammenfügen in-situ im Herzen konfiguriert sind, oder die Vorrichtung einen ersten Teil, der im Wesentlichen das distale Ende (3) umfasst oder aus diesem besteht, und einen zweiten Teil umfasst, der das proximale Ende (4) umfasst oder aus diesem besteht, wobei freie Enden (17) des ersten und des zweiten Teils zum Anliegen in-situ im Herzen konfiguriert sind, wobei der erste Teil wahlweise das distale Ende (3) und den flexiblen Schlauch (2) umfasst und der zweite Teil wahlweise im Wesentlichen aus dem proximalen Ende (4) besteht oder wobei der erste Teil wahlweise das distale Ende (3) und einen ersten Abschnitt des flexiblen Schlauchs (2) und der zweite Teil wahlweise das proximale Ende (4) und einen zweiten Abschnitt des flexiblen Schlauchs (2) umfasst oder wobei freie Enden (17) des ersten und des zweiten Abschnitts des flexiblen Schlauchs wahlweise zum Anliegen in-situ im Herzen konfiguriert sind, oder

die Vorrichtung ein Strukturelement (10) aus Draht und eine biokompatible Verschlusshülse umfasst, die innen oder außen an dem Strukturelement aus Draht angeordnet ist, wobei das Strukturelement aus Draht ein Formgedächtnismaterial oder mehrere umlaufende und radial expandierbare Drahtstreben (10) umfasst, oder die Vorrichtung biologisch abbaubar ist oder die Vorrichtung für ein radiales Zusammenziehen und Einziehen in einen Extraktionskatheter konfiguriert ist oder

ein distales Ende (3) der Vorrichtung einen Verankerungsmechanismus umfasst, der eine Außenhülse (61), die zur Axial- oder Drehbewegung in Bezug auf eine Innenhülse (62) mit der Innenhülse wirkverbunden ist, und ein radial expandierbares Verankerungselement (63) umfasst, das so mit der Außen- und der Innenhülse verknüpft ist, dass eine Axial- oder Drehbewegung der Außenhülse in Bezug auf die Innenhülse bewirkt, dass das radial expandierbare Verankerungselement radial nach außen expandiert, wobei das radial expandierbare Verankerungselement (63) wahlweise an einem Befestigungspunkt an der Innenhülse (62) befestigt ist und die Außenhülse (61) eine Öffnung zum Aufnehmen eines distalen Endes des Verankerungselements aufweist, wobei die Öffnung proximal zum Befestigungspunkt liegt.

15. System, das eine Shunt-Vorrichtung nach einem der Ansprüche 4 bis 8 und eine entfernt gelegene Vorrichtung mit einem Drahtlos-Empfänger, der so konfiguriert ist, dass er Daten zu dem oder jedem erfassten Parameter empfängt, und einer Anzeige zum Anzeigen der Daten und/oder einer Einführvorrichtung (25, 30, 40) umfasst, die zum perkutanen oder transapikalen Einführen der Shunt-Vorrichtung (1) an einer Ziellokalisation im Herzen/in den Blutgefäßen konfiguriert ist.

## Revendications

1. Dispositif de dérivation implantable (1) configuré pour dériver du sang de l'oreillette gauche (C) du cœur à travers une ouverture (J) dans la paroi septale auriculaire (H), le dispositif comprenant un tube (2) configuré pour un ajustement radial entre une configuration de délivrance contractée appropriée pour une délivrance dans un cathéter de délivrance et une configuration déployée développée de manière radiale, le tube ayant une lumière traversante, une extrémité distale (3), et une extrémité proximale (4) configurée pour enjamber une ouverture dans une paroi septale auriculaire et s'ancrer à la paroi, **caractérisé en ce que** l'extrémité distale (3) du tube est configurée pour s'ancrer au sein de la veine azygos et entrer en prise avec la veine azygos de manière étanche aux fluides de sorte que le dispositif (1) est configuré pour dériver du sang de l'oreillette gauche (C) du cœur à la veine azygos (G).

2. Dispositif de dérivation implantable (1) selon la revendication 1, dans lequel l'extrémité distale (3) du dispositif est configurée pour un développement supérieur dans l'ostium de la veine azygos, pour ancrer l'extrémité distale du dispositif dans l'ostium de la veine azygos et créer une connexion étanche aux fluides entre le dispositif de dérivation et la veine azygos.

3. Dispositif de dérivation implantable (1) selon la revendication 1 ou 2, dans lequel le tube (2) est flexible et comprend un élément de fil structurel approprié pour maintenir la perméabilité du dispositif et une gaine d'occlusion biocompatible configurée pour empêcher une fuite de fluide hors du dispositif.

4. Dispositif de dérivation implantable selon l'une quelconque des revendications précédentes, dans lequel le dispositif comprend un capteur pour détecter un paramètre de sang au sein ou à proximité du dispositif de dérivation, dans lequel le capteur est facultativement configuré pour détecter la pression sanguine.

5. Dispositif de dérivation implantable selon la revendication 4, dans lequel le capteur comprend un module de communication sans fil configuré pour envoyer sans fil des signaux du capteur à un emplacement distant.

6. Dispositif de dérivation implantable selon la revendication 4 ou 5, comprenant un deuxième capteur.

7. Dispositif de dérivation implantable selon la revendication 6, dans lequel le capteur est configuré pour détecter un paramètre de sang dans l'oreillette gauche et le deuxième capteur est configuré pour détecter un paramètre de sang dans l'oreillette droite.

8. Dispositif de dérivation implantable selon la revendication 7, dans lequel le capteur est configuré pour détecter la pression sanguine dans l'oreillette gauche et le deuxième capteur est configuré pour détecter la pression sanguine dans l'oreillette droite.

9. Dispositif de dérivation implantable selon l'une quelconque des revendications précédentes, dans lequel le dispositif comprend une valve, dans laquelle la valve est facultativement configurée pour commander le flux sanguin de droite à gauche ou de gauche à droite, ou le passage d'un thrombus jusque dans l'oreillette gauche.

10. Dispositif de dérivation implantable selon la revendication 9, dans lequel la valve est configurée pour être installée à postériori sur le dispositif de dérivation in vivo ou ex vivo.

11. Dispositif de dérivation implantable selon l'une quelconque des revendications précédentes, dans lequel l'extrémité proximale (4) comprend deux sections de bride de retenue capables de développement espacées de manière axiale (5) configurées pour un développement de chaque côté d'une paroi septale auriculaire (H) pour ancrer l'extrémité distale du dispositif.

12. Dispositif de dérivation implantable selon la revendication 11, dans lequel les brides de retenue sont espacées de manière axiale l'une de l'autre d'une distance approximativement égale à une épaisseur de la paroi septale auriculaire (H) où l'ouverture (J) est située.

13. Dispositif de dérivation implantable selon l'une quelconque des revendications précédentes, dans lequel le dispositif est capable d'auto-développement.

14. Dispositif de dérivation implantable selon la revendication 1, dans lequel :

   le dispositif est préformé pour prendre une forme incurvée lors du déploiement ou
   le dispositif est modulaire et fourni en deux parties ou plus (15, 16) configurées pour un assemblage in situ dans le cœur ; ou
   le dispositif comprend une première partie comprenant ou consistant essentiellement en l'extrémité distale (3), et une deuxième partie comprenant ou consistant en l'extrémité proximale (4), dans lequel les extrémités libres (17) des première et deuxième parties sont configurées pour une entrée en prise in situ dans le cœur,
   dans lequel la première partie comprend facultativement l'extrémité distale (3) et le tube flexible (2), et la deuxième partie consiste facultativement essentiellement en l'extrémité proximale (4), ou
   dans lequel la première partie comprend facultativement l'extrémité distale (3) et une première section du tube flexible (2), et la deuxième partie comprend facultativement l'extrémité proximale (4) et une deuxième section du tube flexible (2), ou dans lequel les extrémités libres (17) des première et deuxième sections du tube flexible sont facultativement configurées pour une entrée en prise in situ dans le cœur ; ou

le dispositif comprend un élément de fil structurel (10) et une gaine d'occlusion biocompatible disposée à l'intérieur ou à l'extérieur de l'élément de fil structurel, dans lequel l'élément de fil structurel comprend un matériau à mémoire de forme ou dans lequel l'élément de fil structurel comprend une pluralité d'entretoises de fil circonférentielles et capables de développement de manière radiale (10) ; ou

le dispositif est biodégradable ; ou

le dispositif est configuré pour une contraction radiale et une rétraction jusque dans un cathéter de retrait ; ou

une extrémité distale (3) du dispositif comprend un mécanisme d'ancrage comprenant un manchon externe (61) connecté de manière fonctionnelle à un manchon interne (62) pour un mouvement axial ou de rotation par rapport au manchon interne, et un élément d'ancrage capable d'extension de manière radiale (63) associé aux manchons externe et interne de sorte que le mouvement axial ou de rotation du manchon externe par rapport au manchon interne amène l'élément d'ancrage capable d'extension de manière radiale à s'étendre de manière radiale vers l'extérieur, facultativement dans lequel l'élément d'ancrage capable d'extension de manière radiale (63) est attaché au manchon interne (62) au niveau d'un point d'attache, et le manchon externe (61) inclut une ouverture pour la réception d'une extrémité distale de l'élément d'ancrage, dans lequel l'ouverture est située de manière proximale par rapport au point de fixation.

15. Système comprenant un dispositif de dérivation selon l'une quelconque des revendications 4 à 8 et un dispositif éloigné comprenant un récepteur sans fil configuré pour recevoir des données concernant le ou chaque paramètre détecté et un affichage destiné à afficher les données et/ou un dispositif de délivrance (25, 30, 40) configuré pour la délivrance percutanée ou transapicale du dispositif de dérivation (1) à un emplacement cible dans le cœur/le système vasculaire.

Figure 1

Figure 2

Figure 3B

Figure 3C

Figure 3A

Figure 4A

Figure 4B

Figure 4C

EP 3 968 852 B1

Figure 5B

Figure 5A

Figure 6

Figure 7

Figure 8

Figure 9B

Figure 9C

Figure 9D

Figure 9E

Figure 9F

Figure 9G

Figure 9A

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6468303 B, Amplatz
- US 20050165344 A, Dobak, III
- US 6562066 B
- US 2008109069 A
- US 2017281339 A
- US 64648303 B
- US 20170113026 A
- US 20180263766 A